# FASCICULE DE BREVET EUROPEEN

(11) **EP 4 188 113 B1**
(45) Date de publication et mention de la délivrance du brevet: **17.12.2025**
(21) Numéro de dépôt: 21758322.8
(22) Date de dépôt: 28.07.2021
(51) Int. Cl.: A23K 20/158, A23K 50/75, A23K 50/80, A61K 31/19, A01N 25/00, A01N 37/00

(54) **COMPOSITION ANTIPARASITAIRE ET SES UTILISATIONS**
ANTIPARASITÄRE ZUSAMMENSETZUNG UND IHRE VERWENDUNGEN
ANTI-PARASITIC COMPOSITION AND USES THEREOF

(30) Priorité: 28.07.2020 EP 20305865
(43) Date de publication de la demande: 07.06.2023
(73) Titulaire: MIXSCIENCE, 35170 Bruz (FR)
(72) Inventeur: VAN DER WEEËN, Pieter, 9000 Gent (BE); FRAGNET, Bruno, 60280 Margny-lès-Compiègne (FR); CASTIER, Julie, 35172 BRUZ CEDEX (FR); FROUEL, Stéphane, 35172 BRUZ CEDEX (FR)
(74) Mandataire: LLR
(86) Numéro de dépôt international: PCT/EP2021/071218
(87) Numéro de publication internationale: WO 2022/023449

(56) Documents cités:
- CN-A- 101 422 209
- JP-B2- 3 480 566
- US-A- 5 462 967
- NORITAKA HIRAZAWA ET AL: "Antiparasitic effect of medium-chain fatty acids against the ciliate Cryptocaryon irritans infestation in the red sea bream Pagrus major", AQUACULTURE, vol. 198, no. 3-4, 1 July 2001 (2001-07-01), Amsterdam, NL, pages 219 - 228, XP055748589, ISSN: 0044-8486, DOI: 10.1016/S0044-8486(01)00503-8

## Description

L'invention concerne un produit antiparasitaire, une composition antiparasitaire comprenant ce produit et leurs utilisations.

Les maladies parasitaires ou parasitoses sont des maladies liées à des parasites qui viennent infecter un organisme hôte animal ou humain.

De telles maladies sont particulièrement néfastes dans les élevages et ont pour conséquence une diminution significative de la performance pouvant conduire à une forte mortalité de l'hôte soit directement soit le plus souvent indirectement (l'affaiblissement de l'hôte par le parasite étant si important que cela sert de voie d'entrée pour d'autres pathogènes de types bactériens et/ou viraux).

Toutes les espèces aquacoles commerciales telles que les salmonidés, les crevettes, les poissons marins, le tilapia et les poissons-chats de type pangasius souffrent d'une variété d'infections par des parasites qui causent souvent d'importantes pertes économiques. Les parasites de branchies et les douves de la peau causent des problèmes croissants dans les piscicultures, telles que *Sparycotyle crisophrii* chez la daurade méditerranéenne *Sparus aurata,* ou les Gyrodactilidés chez le tilapia dans le monde entier. Une grande diversité d'ectoparasites protozoaires infeste les poissons d'eau douce, notamment les parasites associés aux maladies dites des taches blanches, *Trichodina spp,* ... Les endoparasites tels que les vers digéniens, les acanthocéphales, les coccidiens, les mixosporidiens et les microsporidiens attaquent le système digestif et les organes internes. Récemment, en élevages de crevettes, des parasites intestinaux tels que les grégarines et les microsporidiens comme *Enterocytozoon hepatopenaei* (EHP) sont revenus au centre des préoccupations des éleveurs. Connus depuis longtemps pour infester les intestins des crevettes d'élevage, ces parasites font l'objet d'une attention nouvelle de la part des professionnels. Il en est de même pour les ectoparasites tels que le pou du saumon, *Lepeophtheirus salmonis,* qui est une espèce de copépodes parasites de la famille des Caligidae. En effet, s'ils ne causent pas de mortalités massives, ces parasites entraînent des retards de croissance de plus en plus pénalisants et favorisent potentiellement l'émergence de pathogènes secondaires notamment bactériens et viraux.

Chez les animaux d'élevage terrestres, on dénombre également de nombreuses maladies parasitaires.

La coccidiose est parmi les maladies parasitaires les plus fréquentes chez les volailles. Causée par des espèces spécifiques aux oiseaux du genre *Eimeria,* elle peut prendre de nombreuses formes et se rencontre dans le monde entier et dans tout type d'élevage avicole. Le diagnostic clinique est difficile, du fait des symptômes peu spécifiques, variés et de co-infections fréquentes. La coccidiose se traduit souvent par une frilosité des volailles, une baisse d'appétit, un retard de croissance, une apathie ou encore des diarrhées. Tous ces symptômes sont le résultat de dommages intestinaux, qui induisent des pertes économiques significatives pour l'éleveur. D'autant plus que la coccidiose est très contagieuse. Les lésions, si elles sont bien marquées, peuvent être caractéristiques.

Autres maladies parasitaires aviaires fréquentes, les phtiriases, ou maladies des poux, sont des affections causées par des parasites venant par intermittence sur les volailles ou y demeurant en permanence. Le pou rouge ou dermanysses des volailles ou encore *Dermanyssus* est l'un des principaux ectoparasites aviaires. Ces parasites hématophages, particulièrement communs chez la poule pondeuse, sont des vecteurs d'autres agents pathogènes. Les poules infestées sont agitées, affaiblies, amaigries et leur rythme de ponte en est également affecté. On dénombre de nombreux autres types de poux chez les volailles, parmi lesquels *Menacanthus stamineus, Menopon gallinae, Cuclotogaster heterographus* ou *Liperus caponis.*

D'autres ectoparasites aviaires, tels que les tiques et les sarcoptiformes, agents de la gale *(Cnemidocoptes mutans et Cnemidocoptes laevis* notamment) entraînent eux aussi des réductions de performances des animaux et donc des pertes économiques pour l'éleveur.

Afin de lutter contre ces parasitoses, plusieurs approches ont été développées.

L'approche traditionnelle de la prévention et de la lutte contre les parasites des espèces aquatiques, basée sur l'utilisation de produits chimiques et de certains produits thérapeutiques une fois que l'épidémie de parasites est détectée, est de plus en plus entravée par le développement d'une résistance et les restrictions notamment réglementaires et sociétales croissantes sur l'utilisation de tels produits chimiques. Parmi ces produits, on retrouve notamment le bronopol, la chloramine T, le vert de malachite, le sulfate de cuivre, le dimetridazole, le formol, la formaline, le métrofinate, le métronidazole, le niclosamide, le permanganate de potassium, ainsi que de nombreux vermifuges utilisés en médecine humaine. L'utilisation de ces substances, très controversées, posent de nombreuses questions sociétales quant à l'innocuité de ces produits pour l'animal traité, l'environnement et le consommateur. Ces composés sont en effet potentiellement cancérogènes, mutagènes et tératogènes, ce qui posent de nombreux doutes quant à la pérennité de leur utilisation.

En élevage aviaire, face aux enjeux économiques, le principal moyen de lutte contre la coccidiose reste aujourd'hui en France, et plus généralement en Europe, l'utilisation de coccidiostatiques administrés via l'aliment. Cependant, leur utilisation continue et routinière a entraîné l'émergence de sérotypes *d'Eimeria* résistants. De plus, les anticoccidiens utilisés sont potentiellement toxiques pour l'animal traité (notamment chez la dinde où la toxicité de ces molécules est plus forte que chez le poulet), ainsi que pour les animaux tiers.

En élevage aviaire, la prévention passe aussi par l'utilisation de la vaccination : des vaccins vivants sont enregistrés et commercialisés en France. Ceux-ci sont basés sur des souches précoces des espèces majeures de coccidies et permettent de favoriser l'établissement de l'immunité chez les animaux vaccinés. La vaccination donne de bons résultats et l'utilisation de ces vaccins est maintenant répandue sur des productions à haute valeur économique (poulets labels, poules pondeuses, futures reproductrices, etc.) qui justifient ce coût de prophylaxie. Cette alternative pose cependant des problèmes aux éleveurs. En effet, les souches vivantes, quelle que soit leur virulence, doivent se répliquer dans les cellules hôtes épithéliales pour conférer à l'organisme une immunité active. Cette phase de multiplication entraîne une plus grande sensibilité de l'animal avec de possibles baisses de performance, une prédisposition du tractus intestinal à des infections bactériennes gastro-intestinales opportunistes telles que l'entérite nécrotique, des fientes humides et une augmentation des lésions aux pattes.

Le contexte réglementaire de plus en plus contraignant et la prise de conscience générale quant à la qualité sanitaire des aliments rendent indispensable le développement d'alternatives à l'utilisation des produits chimiques, notamment des anticoccidiens, qui soient mieux acceptées par le consommateur, tout en restant économiques pour l'éleveur.

Plus globalement, il est important de maintenir une bonne santé intestinale chez l'animal, afin d'assurer un fonctionnement efficace de la première barrière immunitaire et des mécanismes d'absorption des nutriments.

Ces dernières années ont vu émerger dans l'art antérieur l'utilisation d'acides gras pour prévenir et/ou traiter les parasitoses.

Par exemple, Noritaka Hirazawa et al. (Aquaculture, Volume 198, Issues 3-4, 2 July 2001, Pages 219-228) ont décrit l'utilisation d'acide octanoïque ou acide caprylique (C8) pour diminuer les ectoparasites du genre *Cryptocaryon* au niveau des branchies et des yeux de la daurade *Pagrus major.*

On peut citer également les travaux de Timbermont et al. (Avian Pathology, avril 2010, 39(2),117-121) qui décrivent l'utilisation d'acide butyrique, seul ou en association avec un mélange d'acides gras libres C6 à C12 chez le poulet de chair. Il a été observé un effet additionnel ou synergique de l'acide butyrique avec les acides gras à chaine moyenne sur l'entérite nécrotique, celui-ci ayant certainement une action à la fois sur la bactérie *Clostridium perfringens* elle-même et sur les lésions *Eimeria,* un facteur prédisposant à l'infection bactérienne, permettant ainsi de gérer de manière optimale la pathologie.

On connait en outre la demande US5462967A qui décrit une composition comprenant un mono-, di et/ou triglycéride d'acide hexanoïque et son utilisation médicale pour le traitement des coccidioses. Ce document, qui décrit une composition comprenant des acides gras en C6, C8, C10 et C12, ne donne aucune information quant à la quantité totale d'acide hexanoïque par rapport à la masse totale des autres acides gras.

Le brevet JP3480566B2 décrit une composition comprenant un mono-, di et/ou triglycéride d'acide hexanoïque et son utilisation médicale en tant qu'antiparasitaire. Ici encore, la proportion de C6 n'est pas précisée par rapport à celle des autres acides gras estérifiés. Toutefois, il convient de trouver de nouvelles solutions pour prévenir et traiter les parasitoses.

L'article de Noritaka Hirazawa et al. Aquaculture (2021), vol 198, no. 3-4, pages 219-228 étudie l'effet des acides gras à chaîne moyenne (C6 à C10) sur l'infection des dorades rouges par *Cryptocaryon irritans.* Les résultats montrent que l'acide octanoïque (C8), les acides gras en C2 et C4 n'ayant pas d'effet sur la mortalité du parasite, les acides gras en C6 ou C7 un faible effet à 0,5mM, et les acides gras en C8, C9 et C10 ont un effet dès 0,5mM. Des tests in vivo réalisés à partir de l'acide octanoïque (C8) montrent que cet acide gras semble être le meilleur antiparasitaire.

L'un des buts de l'invention est de pallier les manques de l'art antérieur.

Un autre but de l'invention est de fournir des compositions capables de prévenir et traiter les parasitoses de manière large en élevage, à la fois en élevage avicole et en élevage aquacole.

Il est décrit ici un produit comprenant ou consistant essentiellement en au moins
- un monoglycéride d'un ou plusieurs acide(s) gras, ou
- un diglycéride d'un ou plusieurs acide(s) gras, ou
- un triglycéride d'un ou plusieurs acide(s) gras, ou
- un mélange de ceux-ci,

ledit ou lesdits un ou plusieurs acide(s) gras étant composé(s) d'au moins 30% en masse d'acide hexanoïque, ce pourcentage étant exprimé en masse par rapport à la masse totale d'acides gras,
en association avec une diatomite,
notamment pour son utilisation en tant que médicament.

L'invention concerne une composition consistant en :
- de 10 à 50% en masse d'au moins un support, et
- de 35 à 90% en masse d'un produit en association avec une diatomite, ledit produit consistant essentiellement en au moins
- un monoglycéride d'un ou plusieurs acide(s) gras, ou
- un diglycéride d'un ou plusieurs acide(s) gras, ou
- un triglycéride d'un ou plusieurs acide(s) gras, ou
- un mélange de ceux-ci,

ledit ou lesdits un ou plusieurs acide(s) gras étant un mélange d'acides gras comportant de 4 à 8 atomes de carbone
les pourcentages étant exprimés en masse par rapport à la masse totale de la composition,
ledit ou lesdits un ou plusieurs acide(s) gras étant composé(s) d'au moins 30% d'acide hexanoïque, ce pourcentage étant exprimé en masse par rapport à la masse totale d'acide gras.

L'invention repose sur la constatation faite par les inventeurs que l'utilisation de monoglycéride(s) d'acide(s) gras et/ou de diglycéride(s) d'acide(s) gras et/ou de triglycéride(s) d'acide(s) gras, où la proportion d'acide hexanoïque représente au moins 30% en masse par rapport à la masse totale d'acides gras, en association avec une diatomite, présentait de très bons effets dans la lutte ou la prévention des infections parasitaires chez les animaux d'élevage, à la fois dans les élevages avicoles et les élevages aquacoles.

Le produit selon l'invention comprend donc, ou est essentiellement constitué de
- monoglycéride(s) d'acide(s) gras,
- diglycéride(s) d'acide(s) gras,
- triglycéride(s) d'acide(s) gras,
- monoglycéride(s) d'acide(s) gras et diglycéride(s) d'acide(s) gras,
- monoglycéride(s) d'acide(s) gras et triglycéride(s) d'acide(s) gras,
- diglycéride(s) d'acide(s) gras et triglycéride(s) d'acide(s) gras, ou
- monoglycéride(s) d'acide(s) gras, diglycéride(s) d'acide(s) gras et triglycéride(s) d'acide(s) gras
ledit ou lesdits un ou plusieurs acide(s) gras étant composé(s) d'au moins 30% en masse d'acide hexanoïque, ce pourcentage étant exprimé en masse par rapport à la masse totale d'acides gras, en association avec une diatomite, notamment pour une utilisation médicamenteuse.

Dans l'invention, les termes "acide gras" font référence à une molécule présentant une chaîne carbonée acyclique, saturée ou insaturée, comportant entre 4 et 22 atomes de carbone (C4-C22) avec, à une extrémité, un groupement acide (COOH). On distingue les acides gras en fonction du nombre d'atomes de carbone et du nombre de doubles liaisons.

Par « masse totale d'acides gras », on entend dans le cadre de l'invention la masse totale de toutes les molécules d'acides gras estérifiées sur les molécules de glycérol et contenues dans le/les mono, di et/ou triglycéride(s) d'acide(s) gras. Les acides gras libres ne sont donc pas pris en considération dans la « masse totale d'acides gras » telle que définie dans l'invention.

L'acide hexanoïque est un acide gras saturé comportant 6 atomes de carbone (C6), de formule linéaire CH₃(CH₂)₄COOH, également connu sous les appellations : acide hexylique ou acide caproïque. De préférence, ledit ou lesdits un ou plusieurs acide(s) gras est/sont saturé(s).

De préférence, ledit ou lesdits un ou plusieurs acide(s) gras comporte(nt) de 4 à 12 atomes de carbone, plus préférentiellement de 4 à 8 atomes de carbone.

Les mono, di, et tri-glycérides d'acides gras ou mono, di, et tri-acylglycérols, sont respectivement des mono, di et tri-esters pouvant être préparés par estérification entre un glycérol, appelé également 1,2,3-propanetriol ou glycérine, et respectivement 1, 2 et 3 acides gras.

Le terme « monoglycéride », ou « monoacylglycérol » ou « monoacylglycéride », fait référence à un glycérol estérifié par un acide gras. Le terme « diglycéride », ou « diacylglycérol » ou « diacylglycéride », fait référence à un glycérol estérifié par deux acides gras, identiques ou non. Le terme « triglycéride », ou « triacylglycérol » ou « triacylglycéride », fait référence à un glycérol estérifié par trois acides gras, identiques ou non.

Les diatomites ajoutées en association avec le produit selon l'invention ont pour but d'apporter une fonctionnalité mécanique d'abrasivité et de dessication cellulaire afin de compléter l'effet antiparasitaire du produit de mono, di et/ou triglycéride(s) d'acide(s) gras tel que défini ci-dessus. Ces deux ingrédients (produit et diatomites) agissent en combinaison et sont essentiels.

La diatomite est une roche sédimentaire siliceuse composée en majorité de fossiles de microalgues unicellulaires, les diatomées. Elle est également connue sous les appellations : terre de diatomée, kieselguhr, moler et parfois célite ou terre d'infusoires. La différence entre les différentes diatomites disponibles vient de leur composition en oxydes de silicium, de fer, de calcium, de magnésium, d'aluminium, de sodium, de titane, et de potassium principalement, liée au gisement duquel elles sont extraites et du process appliqué sur le minéral.

De manière avantageuse, la diatomite en association avec le produit susmentionné est de la terre de diatomées.

De manière avantageuse, les diatomites susmentionnées sont de la terre de diatomée calcinée et purifiée en silice cristallisée.

De manière avantageuse, l'invention concerne le produit susmentionné, en association avec une diatomite, où ledit ou lesdits un ou plusieurs acide(s) gras sont composé(s) de 30 à 75% en masse d'acide hexanoïque, ces pourcentages étant exprimés en masse par rapport à la masse totale d'acides gras.

Par de « 30 à 75% en masse d'acide hexanoïque » on entend dans l'invention 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74% ou 75%, en masse d'acide hexanoïque.

Avantageusement le produit susmentionné est un mélange de mono, di et/ou triglycéride(s) d'un ou plusieurs acide(s) gras, ledit ou lesdits un ou plusieurs acide(s) gras étant composé(s) d'au moins 30% en masse d'acide hexanoïque, ce pourcentage étant exprimé en masse par rapport à la masse totale d'acides gras, en tant que médicament.

Le mélange de mono, di et/ou triglycéride(s) d'acide(s) gras, se différencie par :
- le type de glycéride, à savoir mono, di et/ou triglycéride(s) ; et/ou
- la nature des acides gras présents dans le ou les mono, di et triglycéride(s).

**Il** est possible que le mélange comporte au moins 95% en masse de glycéride(s) d'acide(s) gras par rapport à la masse totale de glycérides.

Avantageusement, le mélange comporte au moins 60% en masse de monoglycéride(s) d'acide(s) gras par rapport à la masse totale de glycérides.

Par "masse totale de glycérides", on entend la masse totale de toutes les molécules de monoglycérides, diglycérides et triglycérides présentes dans le produit selon l'invention.

Dans le produit avantageux selon l'invention, il y a au moins un monoglycéride, et un di ou un triglycéride d'acide hexanoïque.

Préférentiellement, le produit selon l'invention comporte :
- 60-75% de monoglycéride(s) d'un ou plusieurs acide(s) gras ;
- 24-39% de diglycéride(s) d'un ou plusieurs acide(s) gras ;
- 1-5% de triglycéride(s) d'un ou plusieurs acide(s) gras ;

au moins 30% en masse dudit ou desdits un ou plusieurs acide(s) gras comporte 6 atomes de carbone ;
les % étant des pourcentages en masse exprimés par rapport à la masse totale de mono, di et triglycérides d'acides gras.

Plus particulièrement, le produit selon l'invention comporte :
- 60-75% de monoglycéride(s) d'un ou plusieurs acide(s) gras ;
- 24-39% de diglycéride(s) d'un ou plusieurs acide(s) gras ;
- 1-5% de triglycéride(s) d'un ou plusieurs acide(s) gras ;

ledit ou lesdits un ou plusieurs acide(s) gras comportant de 4 à 8 atomes de carbone, et au moins 30% en masse des acides gras comporte 6 atomes de carbone ;
les % étant des pourcentages en masse exprimés par rapport à la masse totale de mono, di et triglycérides d'acides gras.

Préférentiellement, ledit ou lesdits un ou plusieurs acide(s) gras est/sont constitué(s) d'au moins 35%, plus préférentiellement au moins 40%, plus préférentiellement au moins 45% en masse d'acide hexanoïque par rapport à la masse totale d'acides gras.

Dans le cadre du produit selon l'invention, s'il contient un seul acide gras, i.e. s'il ne contient que de l'acide hexanoïque, engagé dans des liaisons esters avec le glycérol comme mentionné ci-dessus, l'acide hexanoïque représentera au moins 30% en masse par rapport à la masse totale d'acides gras, puisqu'il représentera 100% des acides gras. Le produit selon l'invention comprend ou consiste essentiellement alors en au moins :
- un monoglycéride d'acide hexanoïque,
- un diglycéride d'acide hexanoïque
- un triglycéride d'acide hexanoïque,
- ou un mélange de ceux-ci.

Selon un aspect avantageux de l'invention, le produit selon l'invention comprend au moins un mono, di et/ou triglycéride(s) d'acide hexanoïque, et au moins un mono, di et/ou triglycéride(s) d'au moins un autre acide gras, par exemple un acide gras ayant 8 atomes de carbone. Dans ce cas de produit selon l'invention où deux acides gras sont représentés, l'acide hexanoïque représentera au moins 30% en masse par rapport à la masse totale d'acides gras, et l'autre acide gras représentera jusqu'à 70% en masse par rapport à la masse totale d'acides gras.

Selon un autre aspect avantageux, le produit selon l'invention comprend au moins un mono, di et/ou triglycéride(s) d'acide hexanoïque, et au moins un mono, di et/ou triglycéride(s) d'au moins deux autres acides gras. Par exemple, lesdits au moins deux autres acides gras peuvent être un acide gras ayant 8 atomes de carbone, et un acide gras ayant 10 atomes de carbone. Dans cet exemple de produit où trois acides gras sont présents, l'acide hexanoïque représente au moins 30% en masse par rapport à la masse totale d'acides gras, et la quantité en masse des deux autres acides gras (dans l'exemple, les acides gras en C8 et en C10) représentera jusqu'à 70% en masse par rapport à la masse totale d'acides gras.

L'homme du métier comprend les calculs à réaliser pour les autres combinaisons de produit possibles selon l'invention.

Il est notable que si le produit selon l'invention comprend des mono, di et/ou triglycérides d'acide hexanoïque et au moins un autre acide gras, différents glycérides peuvent coexister, chaque glycéride pouvant comporter des acides gras différents ou identiques.

De manière avantageuse, ledit ou lesdits un ou plusieurs acide(s) gras du produit selon l'invention comprend plusieurs acides gras outre l'acide hexanoïque (sous forme de mono, di et/ou triglycéride(s)), et notamment un ou plusieurs acide(s) gras choisis parmi les acides gras comportant entre 4 et 8 atomes de carbone, c'est-à-dire des acides gras en C4, des acides gras en C5, des acides gras en C7 et des acides gras en C8.

De manière encore plus avantageuse, le produit selon l'invention comprend des mono, di et/ou triglycérides d'acides gras où les acides gras sont un mélange d'acides gras en C4, C6 et/ou C8. Cela signifie alors que dans le produit selon l'invention sont présents des acides gras en C6 (acide hexanoïque) et des acides gras en C4, ou des acides gras en C6 et des acides gras en C8, ou des acides gras en C6, en C4 et en C8.

L'acide gras en C4 est de préférence un acide gras à chaine saturée, de formule linéaire CH₃(CH₂)₂COOH. Cet acide gras en C4 est également connu sous les appellations : acide butanoïque, acide butyrique ou acide butanique.

L'acide gras en C8 est de préférence un acide gras à chaine saturée, de formule linéaire CH₃(CH₂)₆COOH. Cet acide gras en C8 est également connu sous les appellations : acide octanoïque, acide octylique ou acide caprylique.

Le produit selon l'invention est notamment sous forme liquide.

L'invention concerne en outre le produit susmentionné, notamment en association avec une diatomite, en tant que médicament, en particulier en tant que médicament à usage vétérinaire dans le cadre de traitement de pathologies parasitaires survenant chez les animaux, notamment les animaux d'élevage, telles que décrites ci-après ou dans le cadre de la prévention de ces types de pathologies.

Il peut être avantageux que, dans le médicament susmentionné, ledit produit, notamment en association avec une diatomite, soit associé à un véhicule pharmaceutiquement acceptable ou vétérinairement acceptable, selon les normes en vigueur connues de l'homme du métier.

Le véhicule pharmaceutiquement acceptable ou vétérinairement acceptable peut être un simple véhicule sur lequel est déposé le produit selon l'invention mais peut également jouer un rôle galénique spécifique tel que la libération contrôlée lors de l'ingestion du produit, de sorte que les principes actifs seront libérés de manière retardée.

Avantageusement, ledit ou lesdits un ou plusieurs acide(s) gras du produit selon l'invention comprend/comprennent, consiste/consistent essentiellement ou est/sont constitué(s) de :
- de 5 à 25% d'acide butyrique,
- de 30 à 90% d'acide hexanoïque, et
- de 5 à 45% d'acide octanoïque,
les pourcentages étant exprimés en masse par rapport à la masse totale d'acides gras.

De préférence, ledit ou lesdits un ou plusieurs acide(s) gras du produit selon l'invention comprend/comprennent, consiste/consistent essentiellement ou est/sont constitué(s) de :
- de 15 à 25% d'acide butyrique,
- de 40 à 80% d'acide hexanoïque, et
- de 5 à 35% d'acide octanoïque,
les pourcentages étant exprimés en masse par rapport à la masse totale d'acides gras.

Un produit particulièrement préféré comporte ou consiste essentiellement en
- 60-75% de monoglycéride(s) de plusieurs acides gras ;
- 24-39% de diglycéride(s) de plusieurs acides gras ;
- 1-5% de triglycéride(s) de plusieurs acides gras ;

les % étant des pourcentages en masse exprimés par rapport à la masse totale de mono, di et triglycérides d'acides gras, lesdits acides gras étant composés de :
   - 5-25% d'acide butyrique,
   - 30-90% d'acide hexanoïque, et
   - 5-45% d'acide octanoïque ;
les pourcentages étant exprimés en masse par rapport à la masse totale d'acides gras.

L'invention concerne en outre le produit susmentionné, notamment en association avec une diatomite, destiné à être utilisé dans la prévention et/ou le traitement de pathologies causées par des parasites d'animaux, notamment des animaux d'élevage, notamment des animaux aquatiques d'élevage ou d'oiseaux d'élevage.

Cela signifie que que le produit consistant essentiellement en au moins
- un monoglycéride d'un ou plusieurs acide(s) gras, ou
- un diglycéride d'un ou plusieurs acide(s) gras, ou
- un triglycéride d'un ou plusieurs acide(s) gras, ou
- un mélange de ceux-ci,

ledit ou lesdits un ou plusieurs acide(s) gras étant composé(s) d'au moins 30% en masse d'acide hexanoïque, ce pourcentage étant exprimé en masse par rapport à la masse totale d'acides gras,
en association avec une diatomite,
peut être utilisé pour la prévention et/ou le traitement de pathologies causées par des parasites d'animaux, notamment des animaux d'élevage, notamment des animaux aquatiques d'élevage ou d'oiseaux d'élevage.

Un mélange de mono, di et/ou triglycéride(s) d'un ou plusieurs acide(s) gras, ledit ou lesdits un ou plusieurs acide(s) gras étant composé(s) d'au moins 30% en masse d'acide hexanoïque, ce pourcentage étant exprimé en masse par rapport à la masse totale d'acides gras, notamment en association avec une diatomite, peut être utilisé pour la prévention et/ou le traitement de pathologies causées par des parasites d'animaux, notamment des animaux d'élevage, notamment des animaux aquatiques d'élevage ou d'oiseaux d'élevage.

Le produit susmentionné est efficace pour la prévention et le traitement contre des parasites d'animaux d'élevage, et en particulier des parasites d'oiseaux d'élevage ou d'animaux aquatiques d'élevage.

Par parasites, on entend dans l'invention les endoparasites ou pathogènes endoparasitaires ou pathogènes endoparasitoïdaires, et les ectoparasites ou pathogènes ectoparasitaires ou pathogènes ectoparasitoïdaires.

Par endoparasites ou pathogènes endoparasitaires ou endoparasitoïdaires, on entend dans l'invention des parasites vivant et se développant à l'intérieur, en dedans, de leur hôte, au contraire de l'ectoparasite. Les endoparasites peuvent exister sous l'une des deux formes suivantes : les parasites intercellulaires qui infectent les espaces intercellulaires, ou des parasites intracellulaires qui infectent et se développent directement dans des cellules de l'hôte.

Par ectoparasites ou pathogènes ectoparasitaires ou ectoparasitoïdaires, on entend dans l'invention des parasites vivant et se développant à l'extérieur, en dehors de leur hôte (muqueuses externes, peau, phanères, branchies, ouïes, yeux,...), au contraire de l'endoparasite.

Les pathologies et affections causées par les pathogènes endoparasitaires aquatiques suivants entrent dans la portée de l'invention :
*Acantocephalus, Aggregata, Amphibdella, Amyloodinium, Anguillicoloides, Anophryoides, Aphanomyces, Apiosoma, Aspergillus, Batrachochytrium, Benedenia, Bolbophorus, Bolbosoma, Bonamia, Bucephalus, Camallanus, Capriniana, Cardicola, Centrocestus, Ceratonova, Chilodonella, Chloromyxum, Cichlidogyrus, Clinostomum, Cryptobia, Cryptocaryon, Cryptocotyle, Dactylogyrus, Democystidium, Didymocylindrus, Didymocystis, Diphyllobothrium, Diplectanum, Diplostomum, Diplozoon, Echinorhynchus, Eimeria, Enterocytozon, Enterogyrus, Enteromyxum, Enteropsora, Epistylis, Erilepturus, Fusarium, Galactosomum, Gilquina, Gotocotyla, Goussia, Gymnodinium, Gyrodactylus, Haemogregarina, Haplosporidium, Helicometra, Henneguya, Hepatoxylon, Hexamita, Ichthyobodo, Ichthyophonus, Ichthyophthirius, Kabataia, Katsuwonus, Khawia, Kudoa, Lamellodiscus, Margolisiella, Marteilia, Microcotyle, Microphallus, Monobothrium, Myxobolus, Nasicola, Neobenedenia, Nosema, Paradeontacylix, Paramarteilia, Paramoeba, Parvicapsula, Perkinsus, Phoma, Piscinoodinium, Pleistophora, Pomphorhynchus Pricea, Proctoeces, Prosorhynchus, Proteocephalus, Psettarium, Pseudoloma, Saprolegnia, Schyzocotyle, Sparicotyle, Sphaerospora, Sphaerothecum, Spironucleus, Stephanostomum, Tetracapsuloides, Tetrahymena, Thecamoeba, Thelohanellus, Theolohania, Transversotrema, Triaenophorus, Trichodina, Trypanoplasma, Trypanosoma, Tylocephalum, Unitubulotestis, Uronema, Zeuxapta.*

Les pathologies et affections causées par les pathogènes ectoparasitaires aquatiques suivants entrent dans la portée de l'invention : *Acthteres, Argulus, Astacus, Caligus, Ergasilus, Gyrodactylus, Lepeophtheirus, Lernaea, Livoneca, Piscicola, Salmincola.*

Les pathogènes parasitaires qui par définition ont besoin de leur(s) hôte(s) pour vivre sont le plus souvent la porte d'entrée pour d'autres pathogènes plus virulents tels que les bactéries et virus. La plupart des parasites n'ont donc pas une maladie directement associée même si ce n'est pas le cas pour les parasites suivants cités pour exemple et souvent responsables de morbidités importantes sur les espèces aquacoles :
- *Gyrodactylus salaris,* agent ectoparasitaire responsable de la gyrodactylose chez le saumon Atlantique Salmo salar,
- *Trichodina spp,* agent ectoparasitaire responsable de la trichodinose chez plusieurs espèces de poissons, notamment les tilapias,
- *Trypanosoma* et *Trypanoplasma,* agents endoparasitaires responsables de la trypanosomose chez plusieurs espèces de carpes et de salmonidés,
- *Tetracapsuloides bryosalmonae,* agent endoparasitaire responsable de la Proliferative Kidney Disease (PKD) chez plusieurs espèces de salmonidés,
- *Myxobolus cerebralis,* agent endoparasitaire responsable de la Whriling disease (WD) chez plusieurs espèces de salmonidés,
- *Enteromyxum leei,* agent endoparasitaire responsable de l'Enteromyxidiose chez plusieurs espèces de sparidés.

En volaille, l'agent étiologique des coccidioses est un endoparasite protozoaire intracellulaire appartenant le plus souvent au genre *Eimeria.* Il existe plusieurs espèces de coccidies pour chaque espèce aviaire. Les pathologies et affections causées par les pathogènes endoparasitaires aviaires suivants entrent dans la portée de l'invention :
- chez le poulet : *Eimeria acervulina, Eimeria necatrix, Eimeria maxima, Eimeria brunetti, Eimeria tenella, Eimeria mitis, Eimeria praecox* ;
- chez la dinde *: Eimeria meleagrimitis, Eimeria adenoeides, Eimeria dispersa, Eimeria gallopavonis ;*
- chez l'oie : *Eimeria truncata* (pouvant aussi toucher le canard de Barbarie et le cygne), *Eimeria anseris ;*
- chez le canard : *Tyzzeria perniciosa, Eimeria mulardi* (concernant principalement le canard mulard) ;
- chez la pintade : *Eimeria numidia, Eimeria grenieri ;* et
- chez le pigeon : *Eimeria labbeana.*

Les pathologies et affections causées par les pathogènes ectoparasitaires aviaires suivants entrent dans la portée de l'invention *: Dermanyssus, Menacanthus stamineus, Menopon gallinae, Goniodes gigas, Cuclotogaster heterographus, Liperus gallinae, Liperus caponis, Cnemidocoptes mutans, Cnemidocoptes laevis, Echidnophaga gallinacea, Argas persicus, Ixodes, Argas, Ornithodoros.*

On entend par « la prévention et/ou le traitement » la prévention, ou le traitement, ou la prévention et le traitement.

Le produit susmentionné peut être utilisé comme décrit ci-dessus, c'est-à-dire pour son utilisation en tant que médicament, ou pour la prévention et/ou le traitement susmentionné,
en association avec des diatomites, notamment de la terre de diatomées.

De Le produit susmentionné et les diatomites susmentionnées peuvent être utilisés simultanément (par exemple sous la forme d'une composition de combinaisons comprenant le produit selon l'invention et les diatomites), séparément ou de manière étalée dans le temps.

**Il** est également décrit une composition comprenant
- de 10 à 50% d'au moins un support, et
- de 15 à 80% d'un produit comprenant ou consistant essentiellement en au moins
- un monoglycéride d'un ou plusieurs acide(s) gras, ou
- un diglycéride d'un ou plusieurs acide(s) gras, ou
- un triglycéride d'un ou plusieurs acide(s) gras, ou
- un mélange de ceux-ci,

les pourcentages étant exprimés en masse par rapport à la masse totale de la composition,
ledit ou lesdits un ou plusieurs acide(s) gras étant composé(s) d'au moins 30% d'acide hexanoïque, ce pourcentage étant exprimé en masse par rapport à la masse totale d'acides gras.

De par sa composition majoritairement naturelle, ladite composition vise à remplacer l'approche traditionnelle de plus en plus controversée de la lutte contre les parasites des animaux d'élevage, notamment des espèces aviaires et aquatiques, qui se base sur l'utilisation de produits chimiques et de certains produits thérapeutiques une fois que l'épidémie de parasites est détectée. L'impact de la composition selon l'invention sur l'environnement est donc limité si on le compare aux produits chimiques traditionnellement utilisés.

Plus avantageusement, le produit susmentionné de la composition définie ci-dessus en association avec la diatomite mentionnée plus haut représentent tous deux (produit et diatomite) en masse de 35 à 76% par rapport à la masse totale de la composition.

Cela signifie que le produit en association avec la diatomite représente 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89% ou 90% en masse par rapport à la masse totale de la composition. De préférence, cela signifie que le produit en association avec la diatomite représente 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75% ou 76% en masse par rapport à la masse totale de la composition.

Plus avantageusement, la diatomite susmentionnée, qui est associée au produit défini ci-dessus, de la composition définie ci-dessus, représente (la diatomite) de 18 à 50 % en masse par rapport à la masse totale du produit et de ladite diatomite. Cela signifie que la diatomite représente 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% ou 50% en masse par rapport à la masse totale de produit et de diatomite.

Avantageusement, la diatomite de la composition susmentionnée représente de 5 à 45% en masse par rapport à la masse totale de la composition, c'est-à-dire par rapport à la masse totale de support, de produit et de diatomite. Cela signifie que la diatomite représente 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44% ou 45% en masse par rapport à la masse totale de la composition (c'est-à-dire comprenant le support, le produit et la diatomite). De préférence, la diatomite représente 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39% ou 40% en masse par rapport à la masse totale de la composition.

Avantageusement, la diatomite est de la terre de diatomées, ou toute autre diatomite telle que décrite ci-dessus, voire un mélange de celles-ci.

Dans l'invention, ledit au moins un support est choisi parmi la silice, le carbonate de calcium, la silice pyrogène, le stéarate de calcium, le stéarate de magnésium, le sulfate de calcium, le formiate de calcium, la bentonite, la sepiolite (semoulette), le phosphate (mono)calcique, le dextrose, ou encore les coproduits de l'industrie de première transformation de végétaux tels que les bagasses de tagète (résidus fibreux de la fleur de tagète après extraction des pigments), la rafle de maïs ou les grits de maïs, le remoulage de blé, la semoulette de blé, etc..., ou un mélange de ceux-ci.

Dans l'invention, ledit au moins un support permet de stabiliser la composition sous une forme poudre et d'éviter les agglomérats.

Ledit au moins un support est présent à raison de 10 à 50% en masse par rapport à la masse totale de la composition, ce qui signifie qu'il représente 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49% ou 50% en masse par rapport à la masse totale de la composition.

De manière avantageuse, le support est de la silice. La silice susmentionnée fait référence à de l'acide silicique ou orthosilicique, précipité et séché. Cette forme est également connue sous les appellations : silice amorphe, gel de silice ou silice hydratée.

D'une autre manière avantageuse, le support est une rafle ou grit de maïs. La rafle de maïs correspond à l'axe fibreux de l'épi de maïs sur lequel sont implantés les grains de maïs. Broyé, elle peut servir de support de par son importante porosité.

D'une autre manière avantageuse, le support est une sépiolite. La sépiolite est un minéral argileux de structure fibreuse formée de multiples canaux. Cet arrangement cristallin lui confère une surface d'adsorption particulièrement élevée. La forme semoulette de la sépiolite est une poudre très grossière permettant un écoulement facilité lors des manipulations.

D'une autre manière avantageuse, le support est un remoulage de blé. Le remoulage de blé est un co-produit de la meunerie. **Il** est issu de la fabrication de farine de blé tendre. **Il** constitue l'assise protéique de l'enveloppe du blé entier.

La composition susmentionnée comprend également de 15 à 70 % en masse par rapport à la masse totale de la composition, d'un produit susmentionné. Cela signifie que la composition comprend 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69% ou 70% d'un produit susmentionné en masse par rapport à la masse totale de la composition. De préférence, la composition susmentionnée comprend également de 15 à 60 % en masse par rapport à la masse totale de la composition, d'un produit susmentionné. Cela signifie que la composition comprend 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59% ou 60%, d'un produit susmentionné en masse par rapport à la masse totale de la composition.

La composition selon l'invention est telle que le(s)dit(s) acide(s) gras sont composé(s) d'au moins 30% d'acide hexanoïque, ce pourcentage étant exprimé en masse par rapport à la masse totale d'acide gras.

Le produit contenu dans la composition selon l'invention est notamment sous forme liquide.

Les définitions données concernant les acides gras, les mono, di et triglycérides susmentionnées s'appliquent *mutatis mutandis à* la composition susmentionnée.

De manière avantageuse, l'invention concerne la composition susmentionnée, où ledit ou lesdits un ou plusieurs acide(s) gras est/sont un mélange d'acides gras comportant de 4 à 8 atomes de carbone.

De manière avantageuse, dans la composition susmentionnée, sont présents
- des acides gras en C6 (acide hexanoïque) et des acides gras en C4, ou
- des acides gras en C6 (acide hexanoïque) et des acides gras en C8, ou
- des acides gras en C6 (acide hexanoïque), en C4 et en C8.

L'acide gras en C4 est de préférence un acide gras à chaine saturée, de formule linéaire CH₃(CH₂)₂COOH. Cet acide gras en C4 est également connu sous les appellations : acide butanoïque, acide butyrique ou acide butanique.

L'acide gras en C8 est de préférence un acide gras à chaine saturée, de formule linéaire CH₃(CH₂)₆COOH. Cet acide gras en C8 est également connu sous les appellations : acide octanoïque, acide octylique ou acide caprylique.

De manière encore plus avantageuse, l'invention concerne la composition susmentionnée, où ledit ou lesdits un ou plusieurs acide(s) gras du produit de mono, di et/ou triglycérides d'acide(s) gras comprend/comprennent, consiste/consistent essentiellement ou est/sont constitué(s) de :
- de 5 à 25% d'acide butyrique,
- de 30 à 90% d'acide hexanoïque, et
- de 5 à 45% d'acide octanoïque,
les pourcentages étant exprimés en masse par rapport à la masse totale d'acides gras.

Dans cette composition avantageuse, l'acide butyrique sous forme mono, di et/ou triglycérides est présent à raison de 5%, 6%, 7%, 8%, 9%, 10%, 11% 12%, 13%, 14%, 15%, 16%, 17%, 18%, 19%, 20%, 21%, 22%, 23%, 24% ou 25% en masse par rapport à la masse totale d'acides gras.

Comme mentionné précédemment, l'acide hexanoïque sous forme mono, di et/ou triglycérides est présent à raison de 30% ou plus, et notamment à raison de 30%, 31 %, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89% ou 90% en masse par rapport à la masse totale d'acides gras.

Dans cette composition avantageuse, l'acide octanoïque sous forme mono, di et/ou triglycérides est présent à raison de 5%, 6%, 7%, 8%, 9%, 10%, 11% 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44% ou 45% en masse par rapport à la masse totale d'acides gras.

Ces proportions s'appliquent *mutatis mutandis* au produit pour son utilisation susmentionné.

De manière avantageuse, l'invention concerne la composition susmentionnée, où ladite composition comprend en outre jusqu'à 20% de glycérol, le pourcentage étant exprimé en masse par rapport à la masse totale de la composition.

**Il** est préférable que la composition selon l'invention ne comporte pas de glycérol non estérifié avec des acides gras, appelé également glycérol libre. Toutefois, il est tolérable dans la composition selon l'invention qu'une quantité maximale de glycérol libre de 20% en masse par rapport à la masse totale de la composition soit présente.

Avantageusement, la quantité de glycérol non estérifié est inférieur à 10%, notamment 5% par rapport à la masse totale de la composition. **Il** est particulièrement avantageux que la composition ne comprenne pas de glycérol.

Le glycérol libre présent dans la composition est un sous-produit de la réaction d'estérification. **Il** est généralement éliminé en fin de synthèse, toutefois des traces peuvent subsister dans la limite susmentionnée (i.e. 20% en masse par rapport à la masse totale de la composition).

Par analogie, la présence de glycérol libre dans la composition susmentionnée s'applique *mutatis mutandis* au produit tel que défini ci-dessus, et au produit tel que défini ci-dessus pour son utilisation telle que définie ci-dessus. Cela signifie que ledit produit ou ledit produit pour son utilisation ne comprend pas plus de 20% de glycérol libre en masse par rapport à la masse dudit produit.

De manière avantageuse, l'invention concerne la composition susmentionnée, ladite composition comprenant en outre de 5 à 45% de diatomites, notamment de la terre de diatomées, le pourcentage étant exprimé en masse par rapport à la masse totale de la composition.

L'invention s'appuie sur l'application combinée d'un produit selon l'invention associé à d'autres ingrédients minéraux, comme les diatomites.

Dans cette composition avantageuse, les diatomites sont présentes à raison de 5%, 6%, 7%, 8%, 9%, 10%, 11% 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44% ou 45% en masse par rapport à la masse totale de la composition.

De manière avantageuse, l'invention concerne la composition susmentionnée, où le produit comprend, consiste essentiellement ou est constitué d'au moins 60% de monoglycéride(s), le pourcentage étant exprimé en masse par rapport à la masse totale dudit produit.

Dans le produit de la composition selon l'invention, il est avantageux que les formes monoglycérides soient prépondérantes par rapport aux formes di ou triglycérides. Aussi, avantageusement, les formes monoglycérides constituent au moins 60% en masse des glycérides du produit selon l'invention, ce qui signifie que les diglycérides et les triglycérides représentent jusqu'à 40% en masse des glycérides du produit.

L'invention concerne en outre les dix-huit compositions telles que décrites dans les tableaux 2 et 3, présentés ci-après.

**Il** est décrit un procédé de préparation d'un produit comprenant ou consistant essentiellement en au moins
- un monoglycéride d'un ou plusieurs acide(s) gras, ou
- un diglycéride d'un ou plusieurs acide(s) gras, ou
- un triglycéride d'un ou plusieurs acide(s) gras, ou
- un mélange de ceux-ci,

ledit ou lesdits un ou plusieurs acide(s) gras étant composé(s) d'au moins 30% en masse d'acide hexanoïque, ce pourcentage étant exprimé en masse par rapport à la masse totale d'acides gras,
comprenant une étape de mélange d'acide(s) gras comprenant au moins 30% d'acide hexanoïque, avec du glycérol et de chauffage du mélange obtenu à au moins 160°C. Il s'agit ici d'une estérification, les réactions d'estérification étant bien connues de l'homme du métier.

Le produit de cette estérification est notamment liquide.

Le produit susmentionné peut également être préparé par mélange de mono, di, et/ou triglycéride(s) d'acides gras purs commerciaux. Le mélange est alors réalisé dans les proportions souhaitées, notamment telles que définies ci-dessus. Ici encore le produit résultant est notamment un produit liquide.

**Il** est également décrit un produit de mono, di et/ou triglycéride(s) d'acide(s) gras susceptible d'être obtenu par le procédé susmentionné.

L'invention concerne un procédé de préparation d'une poudre de la composition telle que définie ci-dessus, ledit procédé comprenant
- la mise en contact d'un produit susmentionné liquide, le ou les acide(s) gras étant composé(s) d'au moins 30% en masse d'acide hexanoïque, le pourcentage étant exprimé en masse par rapport à la masse totale d'acides gras, avec au moins un support sous forme de poudre, dans des proportions de 10 à 50% dudit au moins un support, et de 15 à 70% dudit produit, et
- le brassage, notamment homogène, dudit produit liquide avec ledit au moins un support, pour obtenir une poudre de ladite composition.

De manière avantageuse, l'invention concerne un procédé de préparation d'une poudre de la composition telle que définie ci-dessus, ledit procédé comprenant :
le brassage, notamment homogène, d'un produit liquide comprenant ou consistant essentiellement en au moins
   - un monoglycéride d'un ou plusieurs acide(s) gras, ou
   - un diglycéride d'un ou plusieurs acide(s) gras, ou
   - un triglycéride d'un ou plusieurs acide(s) gras, ou
   - un mélange de ceux-ci,
ledit ou lesdits un ou plusieurs acide(s) gras étant composé(s) d'au moins 30% en masse d'acide hexanoïque, ce pourcentage étant exprimé en masse par rapport à la masse totale d'acides gras,
avec au moins un support sous forme de poudre, dans des proportions de 10 à 50% dudit au moins un support, et de 15 à 70% dudit produit liquide, pour obtenir une poudre de ladite composition.

De manière avantageuse, l'invention concerne un procédé de préparation d'une poudre de la composition telle que définie ci-dessus, ledit procédé comprenant
- l'estérification, notamment par chauffage à au moins 160°C, d'un mélange d'acide(s) gras comprenant au moins 30% d'acide hexanoïque avec du glycérol, pour obtenir un produit sous forme liquide,
- le brassage, notamment homogène, d'un produit sous forme liquide issu de l'étape précédente ledit produit étant composé d'au moins 30% en masse d'acide hexanoïque, le pourcentage étant exprimé en masse par rapport à la masse totale d'acides gras, avec au moins un support sous forme de poudre, dans des proportions de 10 à 50% dudit au moins un support, et de 15 à 70% dudit produit liquide, pour obtenir une poudre de ladite composition.

De manière encore plus avantageuse, l'invention concerne un procédé de préparation d'une poudre de la composition telle que définie ci-dessus, ledit procédé comprenant
le brassage d'un produit susmentionné liquide, le ou les acide(s) gras étant composé(s) d'au moins 30% en masse d'acide hexanoïque, le pourcentage étant exprimé en masse par rapport à la masse totale d'acides gras, avec au moins un support sous forme de poudre et des diatomites, dans des proportions de 10 à 50% dudit au moins un support, de 5 à 45% desdites diatomites et de 15 à 70% dudit produit sous forme liquide, pour obtenir une poudre de ladite composition.

De manière avantageuse, l'invention concerne un procédé de préparation d'une poudre de la composition telle que définie ci-dessus, ledit procédé comprenant :
le brassage d'un premier, d'un deuxième et d'un troisième ingrédient, pour obtenir une poudre de ladite composition,
   1- le premier ingrédient étant un produit liquide comprenant ou consistant essentiellement en au moins
      - un monoglycéride d'un ou plusieurs acide(s) gras, ou
      - un diglycéride d'un ou plusieurs acide(s) gras, ou
      - un triglycéride d'un ou plusieurs acide(s) gras, ou
      - un mélange de ceux-ci,

      ledit produit représentant de 15 à 70% en masse,
      ledit ou lesdits un ou plusieurs acide(s) gras étant composé(s) d'au moins 30% en masse d'acide hexanoïque, ce pourcentage étant exprimé en masse par rapport à la masse totale d'acides gras,
   2- le deuxième ingrédient étant une diatomite sous forme de poudre, dans une proportion de 5 à 45% en masse, et
   3- le troisième ingrédient étant au moins un support sous forme de poudre dans une proportion de 10 à 50% en masse,
où les pourcentages sont exprimés en masse par rapport à la masse totale des premier, deuxième et troisième ingrédients.

Le procédé comprend également avantageusement une étape de récupération de la poudre de la composition telle qu'obtenue ci-dessus.

Le brassage est notamment un brassage uniforme et/ou homogène.

L'invention concerne également une composition sous forme de poudre susceptible d'être obtenue par le(s) procédé(s) susmentionné(s).

L'estérification susmentionnée est préférentiellement conduite à une température d'au moins 160°C, plus préférentiellement d'au moins 180°C, telle que 200°C.

Le chauffage est maintenu jusqu'à ce que l'indice d'acide soit inférieur à 5, préférentiellement inférieur à 3 mg de KOH/g.

L'indice d'acide (AV) est défini comme le nombre de milligrammes d'hydroxyde de potassium nécessaires pour neutraliser les fonctions acides présentes dans un gramme d'une substance chimique. Cette valeur, sauf indication contraire, a été mesurée selon la norme AOCS Cd 3d-63.

Un entrainement à la vapeur peut être réalisé à la suite de l'estérification afin d'éliminer les acides gras résiduels.

Un catalyseur d'estérification peut être utilisé, tel l'hydroxyde de calcium ou le stéarate de calcium.

Avantageusement, l'eau formée en cours de réaction est éliminée au fur et à mesure.

Dans un autre aspect, l'invention concerne un produit alimentaire comprenant de 0,001% à 2%, notamment de 0,002% à 1%, d'un produit tel que défini ci-dessus, notamment en association avec une diatomite, ou d'une composition telle que définie ci-dessus,
les pourcentages étant exprimés en masse par rapport à la masse totale du produit alimentaire.

Le produit alimentaire décrit dans l'invention est un aliment destiné à l'alimentation animale.

Par « de 0,001 % à 2% », on entend dans l'invention 0,001%, 0,002%, 0,003%, 0,004%, 0,005%, 0,01%, 0,02%, 0,03%, 0,04%, 0,05%, 0,06%, 0,07%, 0,08%, 0,09%, 0,1%, 0,11%, 0,12%, 0,13%, 0,14%, 0,15%, 0,16%, 0,17%, 0,18%, 0,19%, 0,2%, 0,21%, 0,22%, 0,23%, 0,24%, 0,25%, 0,26%, 0,27%, 0,28%, 0,29%, 0,3%, 0,31%, 0,32%, 0,33%, 0,34%, 0,35%, 0,36%, 0,37%, 0,38%, 0,39%, 0,4%, 0,41%, 0,42%, 0,43%, 0,44%, 0,45%, 0,46%, 0,47%, 0,48%, 0,49%, 0,5%, 0,51%, 0,52%, 0,53%, 0,54%, 0,55%, 0,56%, 0,57%, 0,58%, 0,59%, 0,6%, 0,61%, 0,62%, 0,63%, 0,64%, 0,65%, 0,66%, 0,67%, 0,68%, 0,69%, 0,7%, 0,71%, 0,72%, 0,73%, 0,74%, 0,75%, 0,76%, 0,77%, 0,78%, 0,79%, 0,8%, 0,81%, 0,82%, 0,83%, 0,84%, 0,85%, 0,86%, 0,87%, 0,88%, 0,89%, 0,9%, 0,91%, 0,92%, 0,93%, 0,94%, 0,95%, 0,96%, 0,97%, 0,98%, 0,99%, 1%, 1,01%, 1,02%, 1,03%, 1,04%, 1,05%, 1,06%, 1,07%, 1,08%, 1,09%, 1,1%, 1,11%, 1,12%, 1,13%, 1,14%, 1,15%, 1,16%, 1,17%, 1,18%, 1,19%, 1,2%, 1,21%, 1,22%, 1,23%, 1,24%, 1,25%, 1,26%, 1,27%, 1,28%, 1,29%, 1,3%, 1,31%, 1,32%, 1,33%, 1,34%, 1,35%, 1,36%, 1,37%, 1,38%, 1,39%, 1,4%, 1,41%, 1,42%, 1,43%, 1,44%, 1,45%, 1,46%, 1,47%, 1,48%, 1,49%, 1,5%, 1,51%, 1,52%, 1,53%, 1,54%, 1,55%, 1,56%, 1,57%, 1,58%, 1,59%, 1,6%, 1,61%, 1,62%, 1,63%, 1,64%, 1,65%, 1,66%, 1,67%, 1,68%, 1,69%, 1,7%, 1,71%, 1,72%, 1,73%, 1,74%, 1,75%, 1,76%, 1,77%, 1,78%, 1,79%, 1,8%, 1,81%, 1,82%, 1,83%, 1,84%, 1,85%, 1,86%, 1,87%, 1,88%, 1,89%, 1,9%, 1,91%, 1,92%, 1,93%, 1,94%, 1,95%, 1,96%, 1,97%, 1,98%, 1,99% ou 2%.

Le terme d'aliment destiné à l'alimentation animale prend ici le sens normalement attribué par l'homme de métier. Par exemple, un tel aliment peut être considéré comme des aliments artificiels (c'est-à-dire formulés et fabriqués) pour suppléer ou remplacer des aliments naturels dans l'industrie de la nutrition animale. Ces aliments sont communément produits sous forme de flocon, granulé pressé, granulé extrudé, granulé coupé court, granulé coupé long, crumble, tablette, farine, semoulette ou semoulette calibrée, petite miette, miette grossière ou vermicelle.

Typiquement, un aliment réfère à un aliment artificiel qui est utile à la croissance des animaux d'élevage. Les aliments formulés sont composés de différents ingrédients dans des proportions variées qui se complètent les uns avec les autres pour former un aliment complet qui répond aux besoins nutritionnels de l'espèce cible, notamment aquacole ou aviaire.

Les aliments sont composés de micro et de macro-ingrédients. En général, les ingrédients utilisés à des doses supérieures à 1% sont appelés macro-ingrédients. Les ingrédients utilisés à des doses inférieures à 1% sont appelés micro-ingrédients. Chacune des catégories macro et micro-ingrédients est subdivisée en composants ayant des fonctions nutritionnelles ou des fonctions techniques. Les ingrédients ayant une fonction technique améliorent les qualités physiques de l'aliment pour animaux. Les ingrédients ayant une fonction nutritive apportent aux animaux les nutriments et l'énergie nécessaires à leur croissance, à leur survie, à leur reproduction et à leur métabolisme de base. Ces nutriments sont les nutriments de base à savoir des glucides, sous forme de sucres simples ou complexes, des lipides, notamment sous forme d'acides gras saturés ou non, de stérols, ou de protides comprenant notamment tout ou partie des vingt acides aminés. Les nutriments comprennent également des minéraux et des vitamines.

De manière avantageuse, le produit alimentaire décrit dans l'invention est un aliment aquacole c'est-à-dire destiné à l'alimentation d'animaux aquatiques dans le but de leur fournir les nutriments nécessaires à leur croissance, survie, reproduction et métabolisme de base.

La composition susmentionnée, sous forme de poudre, peut être intégrée à l'aliment complet destiné aux espèces aquatiques à un dosage allant de 1 à 5g/kg d'aliment. En résumé, un des aspects avantageux est d'utiliser une composition susmentionnée à raison de 0.1 à 0.5% de l'aliment final aquacole.

D'une autre manière avantageuse, le produit alimentaire décrit dans l'invention est un aliment pour oiseaux ou aliment pour volailles c'est-à-dire un aliment destiné à l'alimentation des oiseaux d'élevage ou volailles d'élevage dans le but de leur fournir les nutriments nécessaires à leur croissance, survie, production (oeufs), reproduction et métabolisme de base.

Le produit susmentionné, sous forme liquide, peut être intégré à l'aliment complet destiné aux espèces aviaires à un dosage allant de 150 à 600 g/T d'aliment. En résumé, un des aspects avantageux est d'utiliser un produit susmentionné à raison de 0.015 à 0.06% de l'aliment final aviaire.

La composition susmentionnée, sous forme de poudre, peut être intégrée à l'aliment complet destiné aux espèces aviaires à un dosage allant de 500 g/T à 2 kg/T d'aliment. En résumé, un des aspects avantageux est d'utiliser une composition susmentionnée à raison de 0.05 à 0.2% de l'aliment final aviaire.

L'invention repose sur la constatation faite par les inventeurs qu'une dose particulière de la composition susmentionnée, dans un mode d'administration précis, permet de prévenir l'infection par des parasites, notamment des endoparasites, des structures aquacoles ou aviaires.

L'invention concerne également la composition telle que définie ci-dessus, ou le produit alimentaire susmentionné, pour son utilisation en tant que médicament, notamment destiné(e) à être utilisé(e) pour la prévention et/ou le traitement de pathologies causées par des parasites d'animaux, notamment des animaux d'élevage, notamment des animaux aquatiques d'élevage ou d'oiseaux d'élevage.

Dans l'invention, on entend par animaux d'élevage tout animal non sauvage, qui est utilisé dans l'industrie agroalimentaire, ou des animaux domestiques y compris les nouveaux animaux domestiques ou de compagnie. De manière avantageuse, les animaux d'élevage sont des animaux aquatiques ou des oiseaux, notamment des volailles.

Par espèces ou animaux aquatiques, on entend les espèces d'élevage aquacole suivantes : carpes du genre *Carassius, Ctenopharyngodon, Cyprinus, Labeo* et *Hypophthalmichthys,* les tilapias du genre Oreochromis, les Crevettes du genre *Pandalus, Palaemon, Penaeus, Litopenaeus et Macrobrachium,* les Salmonidés du genre Salmo et *Oncorhynchus,* les poissons chat du genre *Clarias, Pangasius et Ictalurus,* les bars du genre *Dicentrachus, Epinephelus et Lates,* les daurades du genre *Sparus, Pagrus, Spondyliosoma et Lithognathus,* les Percidés du genre *Siniperca et Lates.*

La fréquence recommandée d'application de la composition pour les espèces d'élevage aquacole est une utilisation quotidienne ou plus régulière, pendant au moins quatorze jours, dans le cadre de la prévention et le traitement des infections parasitaires des animaux aquatiques élevés dans des structures aquacoles, le mieux étant une application continue sur l'ensemble de la durée d'élevage, les parasites étant potentiellement toujours présents dans l'environnement d'élevage.

Par « pendant au moins quatorze jours » on entend dans l'invention une utilisation de 14 jours, 15 jours, 16 jours, 17 jours, 18 jours, 19 jours, 20 jours, 21 jours, 22 jours, 23 jours, 24 jours, 25 jours, 26 jours, 27 jours, 28 jours, 29 jours, 30 jours ou 31 jours, voire plus. L'homme du métier adaptera l'utilisation selon les besoins prophylactiques de la culture aquacole considérée. En tout état de cause, il est important que l'utilisation soit d'au moins 14 jours.

La composition peut être incluse à l'aliment pendant les stades larvaires, post larvaires, juvéniles ou tout autre stade de croissance de l'animal aquacole.

En l'absence de maladie, la composition décrite dans l'invention est ajoutée à titre préventif dans l'aliment aquacole et au cours du processus de fabrication de cet aliment à une dose allant de 0.1 à 0.5% pendant au moins 14 jours consécutifs. L'intégration de la composition, considérée comme micro-ingrédient se fait au moment du mélange des ingrédients à la dose recommandée avant le processus d'extrusion ou le processus de presse.

Si la maladie est détectée dans l'environnement d'élevage, la composition décrite dans l'invention est ajoutée à titre curatif en dernière minute dans l'aliment aquacole directement par technique de top dressing ou top coating (pulvérisation puis enrobage) sur les granulés extrudés et à une dose allant de 0.1 à 0.5%. La quantité de la composition sous forme de poudre est diluée dans de l'huile selon un rapport poudre:huile de 1:15 puis pulvérisée directement à la surface de l'aliment grâce à une rampe de pulvérisation. Si besoin, les granulés sont ensuite enrobés avec de l'huile animale ou végétale afin d'éviter le relargage de la composition une fois dans l'eau d'élevage des espèces aquatiques.

Par les oiseaux d'élevage, on entend les espèces suivantes : les gallinacés, notamment les dindes, les poules, les pintades, les cailles et les faisans, mais également les canards, les autruches, les pigeons, les perdrix et les oies.

La composition décrite dans l'invention est ajoutée, à titre préventif ou curatif, directement dans l'aliment pour volailles au cours du processus de fabrication de cet aliment, au cours du processus de fabrication d'un prémélange destiné à être intégré en aval à un aliment complet, ou directement par technique de top dressing sur l'aliment au niveau de l'élevage. Il est également possible d'administrer ladite composition aux oiseaux d'élevage via l'eau de boisson.

### Brève description des figures

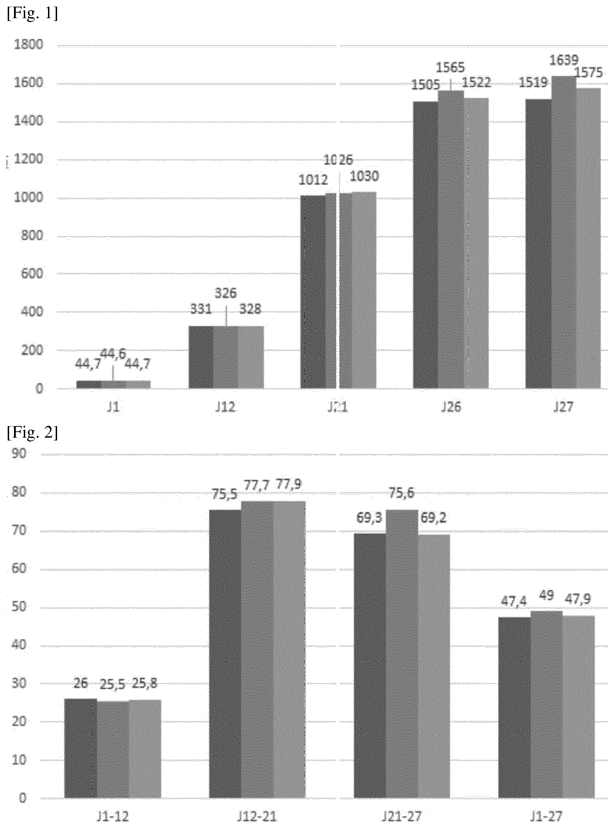

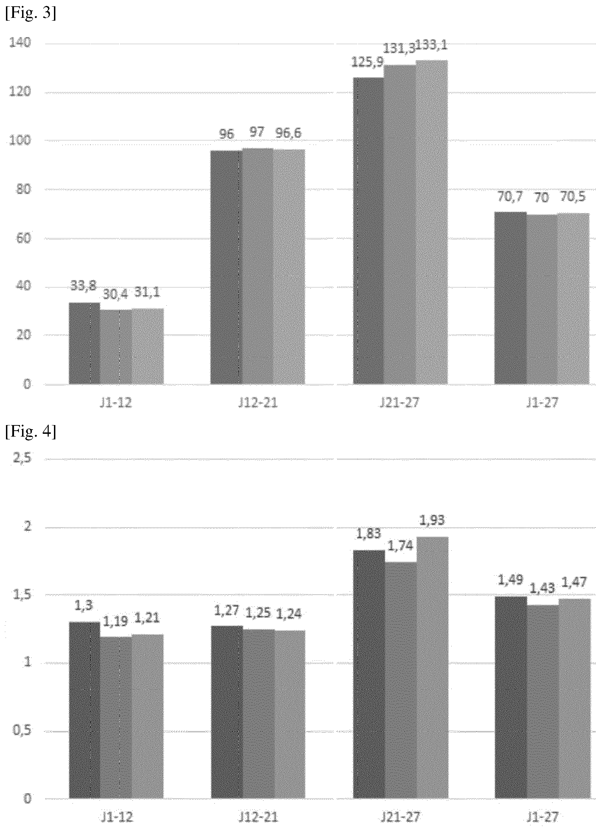

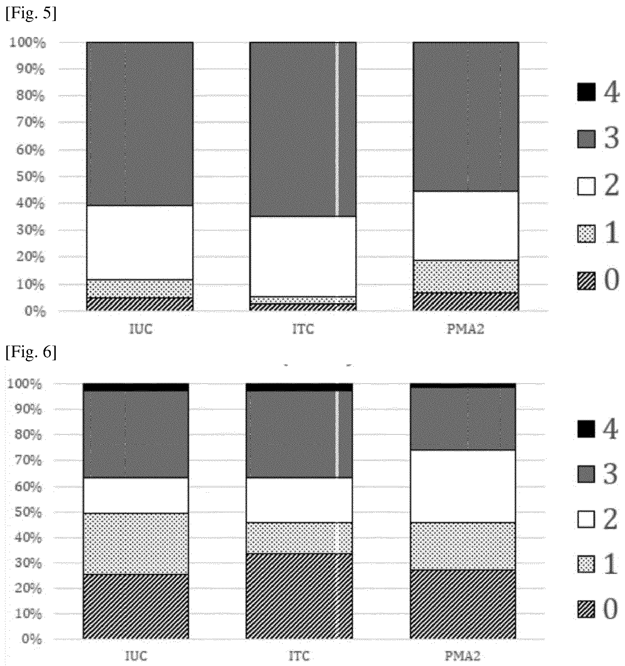

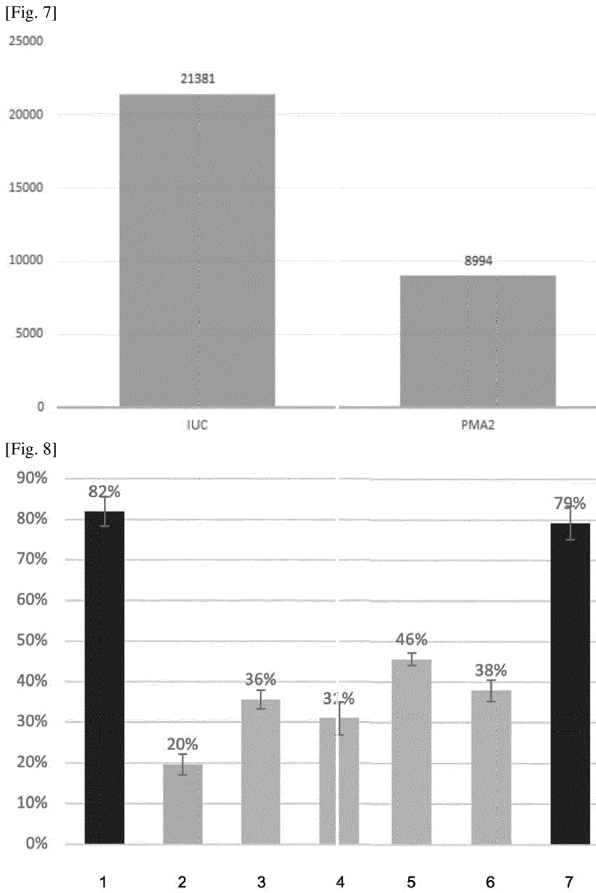

**[****Fig. 1****]**
   **La** **figure 1** est un histogramme montrant le poids moyen de poulets de chair, exprimé en g à J1, J12, J21, J26 et J27, pour chacun des groupes étudiés : lot témoin négatif IUC (gris foncé), lot témoin positif ITC (gris), lot avec apport d'une composition selon l'invention PMA2 (gris clair).
**[****Fig. 2****]**
   **La** **figure 2** est un histogramme montrant le gain moyen quotidien (GMQ) de poulets de chair, exprimé en g/jour, sur les périodes J1 à J12, J12 à J21, J21 à J27 et J1 à J27, pour chacun des groupes étudiés : lot témoin négatif IUC (gris foncé), lot témoin positif ITC (gris), lot avec apport d'une composition selon l'invention PMA2 (gris clair).
**[****Fig. 3****]**
   **La** **figure 3** est un histogramme montrant la consommation moyenne journalière d'aliments (CMJ) de poulets de chair, exprimée en g/jour, sur les périodes J1 à J12, J12 à J21, J21 à J27 et J1 à J27, pour chacun des groupes étudiés : lot témoin négatif IUC (gris foncé), lot témoin positif ITC (gris), lot avec apport d'une composition selon l'invention PMA2 (gris clair).
**[****Fig. 4****]**
   **La** **figure 4** est un histogramme montrant l'efficacité alimentaire de poulets de chair via l'indice de consommation (IC), exprimé en g d'aliment consommé par g de poids vif, sur les périodes J1 à J12, J12 à J21, J21 à J27 et J1 à J27, pour chacun des groupes étudiés : lot témoin négatif IUC (gris foncé), lot témoin positif ITC (gris), lot avec apport d'une composition selon l'invention PMA2 (gris clair).
**[****Fig. 5****]**
   **La** **figure 5** est un histogramme empilé montrant les indices lésionnels coccidiens associés à l'espèce de coccidies *Eimeria acervulina,* mesurés à J26 ou J27 selon la méthode de référence décrite dans Johnson and Reid EXPERIMENTAL PARASITOLOGY 28, 30-36, pour chacun des groupes étudiés : lot témoin négatif IUC, lot témoin positif ITC, lot avec apport d'une composition selon l'invention PMA2.
   Les indices lésionnels sont notés de 0 à 4 : 0 : absence de lésion (hachuré), 1 : lésions en points blancs répartis sur la paroi duodénale uniquement avec moins de 5 lésions au cm² (pointillés), 2 : lésions plus rapprochées mais non coalescentes, jusqu'à 20 cm en-dessous du duodénum, visibles côtés muqueux et séreux (blanc), 3 : lésions jointives, jusqu'au diverticule du sac vitellin, enduit sur la muqueuse, contenu intestinal liquide (gris foncé) et 4 : muqueuse intestinale blanchâtre et très épaissie, exsudat crémeux riche en oocystes (noir).
**[****Fig. 6****]**
   **La** **figure 6** est un histogramme empilé montrant les indices lésionnels coccidiens associés à l'espèce de coccidies *Eimeria maxima,* mesurés sur des poulets de chair à J26 ou J27, selon la méthode de référence décrite dans Johnson and Reid EXPERIMENTAL PARASITOLOGY 28, 30-36, pour chacun des groupes étudiés : lot témoin négatif IUC, lot témoin positif ITC, lot avec apport d'une composition selon l'invention PMA2.
**[****Fig. 7****]**
   **La** **figure** 7 est un histogramme montrant le nombre moyen d'oocystes de coccidies *Eimeria spp.* présents dans 1 g de contenu intestinal prélevé sur des poulets de chairs à J26 ou J27, pour chacun des groupes étudiés : lot témoin négatif IUC (à gauche), lot avec apport d'une composition selon l'invention PMA2 (à droite).
**[****Fig. 8****]**
   **La** **figure 8** est un histogramme montrant le taux moyen de germination des spores *d'Enterocytozoon hepatopenaei* après traitement avec 2 : une composition selon l'invention, 3 : un produit selon l'invention, 4 : un produit enrichi en C8, 5 : de la terre de diatomée seule, et 6 : une composition basée sur le produit selon l'invention mais enrichi en C8. Des spores non traitées (1) ou traitées avec du diméthyl sulfoxide (DMSO) (7) sont utilisées comme contrôles.

### EXEMPLES

### Exemple 1 - Exemple de préparation de produit de mono, di et triglycéride(s) d'acides gras selon l'invention

### Matières premières utilisées :

### Acides gras :

- acide butyrique : Radiacid 0614K commercialisé par Oleon
- acide hexanoïque : Radiacid 0605K commercialisé par Oleon
- acide octanoïque : Radiacid 0608K commercialisé par Oleon
- glycérol : Radia 4810 commercialisé par Oleon

Dans un ballon à 4 cols, surmonté d'un condenseur et équipé d'une sonde de température, d'une agitation mécanique et d'un diffuseur de gaz, 11% d'acide butyrique, 31% d'acide hexanoïque, 12% d'acide octanoïque et 46% de glycérol sont introduits, les pourcentages étant exprimés en masse par rapport à la masse totale des réactifs.

La température du mélange est portée à 160°C et la réaction est stoppée lorsque l'indice d'acide est inférieur à 3 mg de KOH/g.

Un entrainement à la vapeur est ensuite réalisé afin d'éliminer les acides gras n'ayant pas réagi.

Le produit ainsi obtenu comporte :
**A-** 85,3% d'esters de mono, di et triglycérides d'acides gras constitués de :
   - 70,7% de monoglycérides d'acides gras ;
   - 28,0% de diglycérides d'acides gras ;
   - 1,3% de triglycérides d'acides gras ;
   dont les acides gras comprennent :
   - 19,8% d'acide butyrique ;
   - 57,4% d'acide hexanoïque ;
   - 22,8% d'acide octanoïque ;
**B-** 14,7% de glycérol.

### Exemple 2 - Efficacité de la composition selon l'invention pour la prévention des maladies parasitaires en élevages aquacoles

La composition décrite dans l'invention est testée en tant que composition pour la prévention des maladies parasitaires en élevages aquacoles.

Elle est ici testée en conditions expérimentales d'élevage de crevettes blanches *(Penaeus vannamei),* infectées par *Enterocytozoon hepatopenaei* (EHP), responsable de la microsporidiose hépatopancréatique chez la crevette.

Les post larves de crevettes blanches *(Penaeus vannamei)* d'un poids initial individuel moyen de 1,0 g et ayant été diagnostiquées négatives au test de PCR EHP sont réparties dans des réservoirs en plastique de 350 L, remplis avec 250 L d'eau saumâtre et avec une densité de crevettes de 40 individus par bassin (densité équivalente à 160 crevettes/m3). L'eau d'élevage a une salinité de 20 ‰ et une température de 27±1°C.

6 traitements sont appliqués, chacun ayant 5 répliquas :
(1) un aliment spécifique pour la crevette blanche non supplémenté, avec absence d'infection (témoin négatif),
(2) un aliment spécifique pour la crevette blanche non supplémenté, avec infection par *Enterocytozoon hepatopenaei* (EHP) à J14 (témoin challengé),
(3) un aliment spécifique pour la crevette blanche supplémenté avec une composition composée de 75% de silice et 25% de terre de diatomées et appliquée en préventif à 0,2% de l'aliment, avec infection par *Enterocytozoon hepatopenaei* (EHP) à J14 (groupe test (3)),
(4) un aliment spécifique pour la crevette blanche supplémenté avec une composition selon l'invention composée de 43% de silice, 14% de terre de diatomées, et 43% d'un produit selon l'invention, les pourcentages étant exprimés en masse par rapport à la masse totale de la composition, le produit selon l'invention étant constitué de 15% glycérol, 17% C4 sous forme de glycérides, 49% C6 sous forme de glycérides et 19% C8 sous forme de glycérides, les pourcentages étant exprimés en masse par rapport à la masse totale du produit, et appliquée en préventif à 0,35% de l'aliment, avec infection par *Enterocytozoon hepatopenaei* (EHP) à J14 (groupe test (4)) ;
(5) un aliment spécifique pour la crevette blanche supplémenté avec une composition composée de 43% de silice, 14% de terre de diatomées et 43% de monolaurine, et appliquée en préventif à 0,35% de l'aliment, avec infection par *Enterocytozoon hepatopenaei* (EHP) à J14 (groupe test (5)) ;
(6) un aliment spécifique pour la crevette blanche supplémenté avec une composition composée de 30% de silice, 10% de terre de diatomées, 30% d'un produit selon l'invention,et 30% de monolaurine, les pourcentages étant exprimés en masse par rapport à la masse totale de la composition, le produit selon l'invention étant constitué de 15% glycérol, 17% C4 sous forme de glycérides, 49% C6 sous forme de glycérides et 19% C8 sous forme de glycérides, les pourcentages étant exprimés en masse par rapport à la masse totale du produit, et appliquée en préventif à 0,5% de l'aliment, avec infection par *Enterocytozoon hepatopenaei* (EHP) à J14 (groupe test (6)).

Les propriétés antibactériennes de la monolaurine sont bien connue de l'homme du métier.

Pendant toute la période de l'essai d'une durée totale de 64 jours (décomposés en 1 jour d'acclimatation aux bassins expérimentaux suivi de 14 jours sans infection pendant lesquels les traitements sont appliqués dans l'aliment, suivis de 7 jours d'infection expérimentale suivis de 42 jours de post infection), les post larves de crevettes sont nourries 4 fois par jour. La quantité d'alimentation doit être ajustée en fonction de la biomasse estimée et du comportement d'alimentation des crevettes des réservoirs. La consommation d'aliments pendant l'essai est enregistrée chaque semaine.

L'aliment spécifique à la crevette blanche des groupes (1), (2), (3), (4), (5) et (6) est un aliment commercial adapté aux post larves de crevettes blanches sous forme de granulés pressés coulants de taille comprise entre 1,5 mm et 2 mm selon l'âge de la post larve.

Les aliments contenant les traitements sont fabriqués par méthode d'extrusion à froid. Tous les ingrédients (article fin <250 microns) sont mélangés et hydratés (30%) avant d'être extrudés avec un broyeur sous pression. Les aliments extrudés sont ensuite séchés à 50 °C pendant 6 heures. L'humidité finale de l'aliment ne dépasse pas 10%. Les aliments sont ensuite écrasés à la taille attendue (1,5-2 mm de long) puis utilisés dans l'essai. Tous les aliments préparés sont ensuite conservés dans des récipients hermétiques correctement identifiés, réglés à la température ambiante.

Après 14 jours d'élevage dans les conditions décrites précédemment, les crevettes de chacun des bacs, excepté les bacs recevant le traitement (1), sont infectées par *Enterocytozoon hepatopenaei* (EHP) endoparasite responsable de la microsporidiose hépatopancréatique chez la crevette.

L'infection expérimentale se fait par immersion/cohabitation directement dans les bacs d'élevage des crevettes. Des crevettes test infectées par l'EHP ayant une charge connue d'au moins 1,0E + 08 copies par gramme d'hépatopancréas de crevettes sont utilisées pour le challenge. 20 crevettes infectées et 20 crevettes non infectées sont donc placées dans chacun des réservoirs expérimentaux excepté les réservoirs du traitement (1). Les crevettes initialement saines et les crevettes infectées sont séparées par un filet séparateur qui permet le libre-échange de l'eau. Le challenge de cohabitation dure 7 jours et le transfert de parasites se fait via l'eau d'élevage. Les crevettes sont alimentées avec les traitements (1) à (6) pendant toute la durée de l'essai avec les taux d'alimentation suivants : 5% du poids pendant les 14 jours pré-challenge, 3% du poids pendant les 7 jours de challenge et 5% du poids pendant les 42 jours post challenge. La quantité de nourriture est ajustée en fonction du comportement alimentaire des crevettes.

Les crevettes mortes sont retirées des réservoirs quotidiennement. Le taux de survie des crevettes est mesuré à la fin de l'essai tout comme les paramètres de croissance (gain de poids individuel, gain quotidien moyen, taux de croissance spécifique), et d'efficience alimentaire (indice de conversion).

**[Table 1]**

| | **(1)** | **(2)** | **(3)** | **(4)** | **(5)** | **(6)** |
|---|---|---|---|---|---|---|
| **Gain de poids moyen individuel final (g)** | 8,83a | 7,52b | 8,64a | **8,85a** | 7,79b | 7,61b |
| **Indice de conversion final** | 1,16a | 1,60b | 1,61b | **1,38a** | 1,70b | 1,74b |
| **Charge parasitaire (10e5 copies/g d'hépatopancréas)** | 0a | 5964b | 937a | **254a** | 422a | 455a |

Le tableau 1 illustre les paramètres de croissance (gain de poids moyen individuel final (g) et indice de conversion final) et la charge parasitaire finale (10e5 copies d'EHP par gramme d'hépatopancréas) observés chez les juvéniles de crevettes blanches, après infection avec *Enterocytozoon hepatopenaei* (EHP) (excepté pour le groupe (1)). Les valeurs sont présentées sous forme de moyenne (n = 5). Les mêmes lettres (a, b) sur une même ligne indiquent que les différences sont non significatives (p> 0,05). Un indice de conversion bas indique une meilleure conversion de l'aliment et donc une meilleure efficacité alimentaire.

Après 63 jours de culture, après injection de la souche pathogène de *Enterocytozoon hepatopenaei,* les résultats démontrent que la performance et la charge parasitaires des crevettes du groupe témoin challengé (2) sont significativement moins bons que ceux du groupe témoin non challengé (1) validant ainsi les contrôles.

Parmi les groupes expérimentaux testés, le groupe le plus performant est le groupe (4) recevant la composition selon l'invention. Les paramètres de croissance (gain de poids et indice de conversion) et de charge parasitaire finale sont statistiquement non différents du groupe témoin non challengé (1) même si différents numériquement (p<0,05).

Le groupe test (3) comprenant uniquement les ingrédients silice et terre de diatomée performe bien également mais moins que le groupe test (4).

En revanche, les groupes test (5) et (6) sont les groupes qui performent le moins bien. La présence de monolaurine neutralise le potentiel d'efficacité du produit de mono, di et/ou triglycéride(s) d'acide(s) gras selon l'invention, de la silice et de la terre de diatomée.

A l'inverse, dans le groupe test (4), la présence du produit selon l'invention seul (sans monolaurine) renforce l'activité des ingrédients silice et terre de diatomée seuls (groupe test (3)).

### Exemple 3 - Efficacité de la composition selon l'invention en élevage avicole

### I - PROBLEMATIQUE

L'objectif de l'essai est d'évaluer l'impact de la composition selon l'invention sur les performances zootechniques, les lésions intestinales et la charge parasitaire présente dans l'intestin de poulets de chair, après contamination par des coccidies.

### II - MATERIEL ET METHODE

### II.1 - Dispositif expérimental et mise en lot

L'essai a été réalisé dans un dispositif permettant de challenger les animaux par inoculation de micro-organismes pathogènes. 330 poulets mâles Ross 308 ont été allotés à 1 jour, par pesée individuelle, à raison de 22 animaux par parquets d'une surface de 2,4 m², répartis en 3 groupes ou lots de 5 parquets, soit 110 animaux par groupe.

Le challenge suivant a été appliqué afin de favoriser l'apparition d'entérite nécrotique, une infection gastro-intestinale opportuniste favorisée chez les animaux par la présence de coccidies :
- de J12 à J27 : alimentation comportant 5% de protéines de seigle et 10% de farine de poisson,
- à J21 : inoculation de coccidies *(Eimeria acervulina* et *Eimeria maxima)* en une seule fois,
- de J21 à J24 : inoculation de *Clostridium perfringens* 3 fois par jour.

En parallèle de ce challenge, les animaux ont été vaccinés contre la maladie de Newcastle (spray au couvoir puis via l'eau de boisson à J12), la bronchite infectieuse (spray au couvoir, puis via l'eau de boisson à J17) et le Gumboro (à J12 et J17 via l'eau de boisson).

Un même programme alimentaire a été appliqué aux 3 groupes ou lots suivants :
- lot témoin négatif (IUC) : animaux challengés, sans traitement associé ;
- lot témoin positif (ITC) : animaux challengés, avec administration d'amoxicilline (20mg/kg de poids corporel/jour, via l'eau de boisson de J24 à J26) ;
- lot essai avec apport de la composition selon l'invention (PMA2) : animaux challengés, avec apport à hauteur de 0,7 kg/T d'aliment d'une composition selon l'invention dès J0, ladite composition étant constituée avantageusement dans cet essai de 43% de silice, 14% de terre de diatomées, et 43% d'un produit selon l'invention, les pourcentages étant exprimés en masse par rapport à la masse totale de la composition, le produit selon l'invention étant constitué de 15% glycérol, 17% C4 sous forme de glycérides, 49% C6 sous forme de glycérides et 19% C8 sous forme de glycérides, les pourcentages étant exprimés en masse par rapport à la masse totale du produit.

Les animaux ont été abattus à 26 ou à 27 jours d'âge.

### II.2 - Mesures

### II.2.1 - Performances zootechniques

- Mortalité : la mortalité des animaux a été enregistrée sur une base journalière.
- Poids individuel des animaux : pesée individuelle à J1, J12, J21, et J26 ou J27 soit le jour de l'abattage,
- Consommation moyenne journalière d'aliment : mesurée par parquet.

### II.2.2 - Pression coccidienne

- Mesure des indices lésionnels de l'intestin liées aux coccidies *Eimeria acervulina* et *Eimeria maxima* selon l'échelle de Johnson et Reid (1970) le jour de l'abattage (J26 ou J27).
- Dénombrement des oocystes : quantification des oocystes de coccidies de type *Eimeria* spp dans le contenu intestinal prélevé sur les animaux le jour de l'abattage (J26 ou J27), par analyse qPCR (quantitative polymerase chain reaction). L'analyse qPCR est une réaction d'amplification de l'ADN permettant la quantification spécifique des coccidies présentes au niveau de l'intestin des volailles. Cette analyse est effectuée dans un laboratoire indépendant.

### III - Résultats

### III.1 - Poids individuel moyen des animaux

Les résultats sont présentés en **Figure 1****.**

D'après la figure 1, le poids moyen des animaux à la mise en lot (J1) sont équivalents pour les 3 groupes. A J21, au moment de l'inoculation des *Clostridium perfringens* et des coccidies, une différence numérique est observée en faveur du groupe essai (PMA2) : +14g par rapport au groupe témoin positif (ITC) et +18g par rapport au groupe témoin (IUC). A partir de J26, les poids individuels sont significativement différents selon les traitements : le poids individuel moyen des animaux des lots témoin positif (ITC) et essai (PMA2) sont supérieurs de +60g (p < 0.05) et +17g respectivement par rapport au groupe témoin négatif (IUC). A J27, les écarts numériques se creusent : les poids individuels moyens sont de +120g (p < 0.01) pour le groupe témoin positif (ITC) et de +56g pour le groupe essai (PMA2) par rapport au groupe témoin négatif (IUC).

### III.2 - Gain moyen quotidien (GMQ)

Les résultats sont présentés en **Figure 2****.**

Selon la figure 2, les GMQ de la période J1 à J12 sont équivalents entre les 3 groupes d'animaux. Au cours de la période J12-J21, les GMQ des lots témoins positif (ITC) et essai (PMA2) sont numériquement supérieurs à celui du groupe témoin négatif (IUC) respectivement de +2.2 g/j et +2.4 g/j. Sur la période J21-J27, une différence numérique est observée entre les groupes : alors que les GMQ des groupes témoin négatif (IUC) et essai (PMA2) sont équivalents, le GMQ du groupe témoin positif (ITC) est supérieur de 6.3 g/j par rapport au groupe témoin négatif (IUC). Sur la période globale J1-J27, les animaux du groupe témoin positif (ITC) ont des performances de croissance significativement meilleures que celles du lot témoin négatif (IUC) avec une différence de GMQ de +1.6 g/j (p < 0.05), alors que les performances de croissance des animaux essai (PMA2) sont quant à elles numériquement supérieures avec une différence de GMQ de +0.5 g/j par rapport au groupe témoin négatif (IUC).

### III.3 - Consommation moyenne journalière (CMJ)

Les résultats sont présentés en **Figure 3****.**

La figure 3 montre que pour la période J1-J12, la consommation moyenne journalière (CMJ) est significativement inférieure pour les animaux du groupe témoin positif (ITC) avec -3.4 g/j (p < 0.05) par rapport au groupe témoin négatif (IUC). La CMJ du groupe essai (PMA2) est numériquement inférieure de -2.7 g/j comparée au groupe témoin négatif (IUC) pour cette même période. Durant la période J12-J21, les CMJ des groupes témoin positif (ITC) et essai (PMA2) sont numériquement supérieures de +1 g/j et +0.6 g/j respectivement par rapport au groupe témoin négatif (IUC). Au cours de la période J21-J27, une différence numérique est observée sur la CMJ en faveur du groupe essai (PMA2) : +5.4 g/j pour le groupe témoin positif (ITC) et +7.2g/j pour le traitement essai (PMA2). Sur la période globale J1-J27, les CMJ sont globalement équivalentes avec - 0.7 g/j et -0.2 g/j par rapport au groupe témoin négatif (IUC) pour les groupes témoin positif (ITC) et essai (PMA2) respectivement.

### III.4 - Indices de consommation (IC)

Les résultats sont présentés en **Figure 4****.**

Durant la période J1-J12, l'IC le plus performant est celui du groupe témoin positif (ITC) avec -0.11 point par rapport au groupe témoin négatif (IUC). L'IC du groupe essai (PMA2) se rapproche de celui du groupe témoin positif (ITC) avec -0.09 point comparé au groupe témoin négatif (IUC). Au cours de la période J12-J21 jours, l'apport de la composition selon l'invention (groupe essai PMA2) permet d'avoir un IC numériquement meilleur : les IC des groupes témoin positif (ITC) et essai (PMA2) sont respectivement de -0.02 point et -0.03 point par rapport au groupe témoin négatif (IUC). Durant la période finale J21-J27, l'IC le plus performant est celui du groupe témoin positif (ITC) avec -0.09 point comparé au groupe témoin négatif (IUC). Le traitement le moins performant est le PMA2 avec +0.10 point par rapport au groupe témoin (IUC). Sur la période globale 1-27 jours, l'IC du groupe témoin positif (ITC) est significativement inférieur de -0.06 point (p < 0.05) alors que l'IC du groupe essai (PMA2) est numériquement inférieur de -0.02 point, comparé au groupe témoin négatif (IUC).

### III.5 - Mortalité

Sur 110 animaux par groupe, les taux de mortalité s'élèvent à 1%, 6% et 5% respectivement pour les groupes témoin négatif (IUC), témoin positif (ITC) et essai (PMA2). Cette différence numérique est statistiquement non significative et suggère que la mortalité n'est pas un paramètre qui permet de valoriser un traitement plutôt qu'un autre dans cet essai avec challenge *Eimeria.*

### III.6 - Scores lésionnels de Reid

Les résultats sont présentés en **Figures 5** et **6****.**

Selon la figure 5, le groupe essai recevant la composition selon l'invention (PMA2) présente les taux les plus élevés en scores 0 et 1 liés à *Eimeria acervulina,* comparativement aux groupes témoin négatif (IUC) et témoin positif (ITC). Les scores de gravité élevée (supérieur ou égal à 3) se retrouvent, de ce fait, diminués dans le groupe essai (PMA2).

Selon la figure 6, le groupe recevant la composition selon l'invention (PMA2) présente un nombre moins important de scores lésionnels sévères liés *à Eimeria maxima* (score supérieur ou égal à 3), comparativement aux groupes témoin négatif (IUC) et positif (ITC).

### III.7 - Dénombrement des oocystes

Les résultats sont présentés en **Figure 7****.**

L'apport de la composition selon l'invention (groupe essai PMA2) permet de diminuer de 58% la charge parasitaire présente dans le milieu intestinal des poulets par rapport au groupe témoin négatif (IUC).

### IV - Conclusions

Avant l'inoculation de coccidies à J21, l'apport de la composition selon l'invention (PMA2) améliore le poids individuel moyen et le gain moyen quotidien (GMQ) des animaux à consommation moyenne journalière (CMJ) équivalente. L'indice de consommation (IC) du groupe essai (PMA2) est donc le plus performant sur la période 1-21 jours.

Après inoculation de coccidies à J21, l'apport de la composition selon l'invention (PMA2) permet d'améliorer le poids individuel des animaux et la CMJ à GMQ équivalent, comparé au groupe témoin négatif (IUC), ce qui a pour conséquence une dégradation de l'IC.

Durant la période J21-jour de l'abattage (J26 ou J27), le traitement à l'amoxicilline (ITC) est le plus performant avec des poids et GMQ supérieurs aux 2 autres groupes (IUC et PMA2) et une CMJ moyenne située entre ces 2 groupes. L'IC se retrouve ainsi fortement amélioré de 0.09 point par rapport au groupe témoin (IUC) et de 0.19 point par rapport au groupe essai PMA2.

Sur la période globale de l'essai, le gain en performance obtenu au cours de la période J1-J21 pour le groupe essai (PMA2) compense la diminution des performances zootechniques sur la période 21-27 jours. Ainsi, l'apport de la composition selon l'invention permet d'améliorer globalement les performances des poulets par rapport au groupe témoin négatif (IUC) et tend à se rapprocher des performances du groupe traité à l'amoxicilline (ITC).

Par ailleurs, cet essai challengé a permis de mettre en évidence une diminution importante de la présence des oocystes dans le milieu intestinal des animaux recevant la composition selon l'invention. Ceci explique la diminution de sévérité des lésions coccidiennes observées chez les poulets de chair du groupe essai (PMA2) par rapport à celles observées chez les animaux du groupe témoin négatif (IUC) et ceux ayant été traités par antibiotique (ITC).

En conclusion, en situation de challenge renforcé (alimentation riche en protéine + inoculation de *Clostridium perfringens* et de coccidies dans le tube digestif), la composition selon l'invention permet non seulement d'éviter une trop grande diminution des performances zootechniques des poulets de chair, mais empêche l'apparition d'un trop grand nombre de coccidies et de lésions coccidiennes sévères dans le système digestif des animaux, démontrant son effet préventif.

### Exemple 4 - Effets in vitro d'une composition selon l'invention sur la germination et la viabilité de spores d'Enterocytozoon hepatopenaei

L'objectif de cette étude est de tester *in vitro* l'effet d'une composition selon l'invention pour son potentiel à inhiber ou réduire la germination et la viabilité des spores *d'Enterocytozoon hepatopenaei* (EHP).

### I - MATERIEL & METHODES

### I.1 - Design expérimental

Traitements par essai (N=7); Réplicas par essai = 3 et nombre de répétition de l'essai = 2.

Les différents traitements réalisés sont les suivants :
1- Aucun traitement - Spores uniquement dans PBS (Phosphated Buffer Saline),
2- Spores traitées avec une composition selon l'invention : esters glycériques de C4/C6/C8 où le C6 (acide hexanoïque) représente plus de 30% en masse des acides gras en présence de support (silice) et de terre de diatomées,
3- Spores traitées avec le produit selon l'invention : esters glycériques de C4/C6/C8 ou le C6 (acide hexanoïque) représente plus de 30% en masse des acides gras),
4- Spores traitées avec le produit enrichi en C8 : esters glycériques de C4/C6/C8 où le C6 (acide hexanoïque) représente 25% en masse des acides gras et le C8 (acide octanoïque) représente 51% en masse des acides gras, les ratios des mono-di- et triglycérides étant conservés par rapport au traitement 3.
5- Spores traitées avec de la terre de diatomées seule,
6- Spores traitées avec une composition selon l'invention modifiée et enrichie en C8 : esters glycériques de C4/C6/C8 où le C6 (acide hexanoïque) représente 25% en masse des acides gras et le C8 (acide octanoïque) représente 51% en masse des acides gras, les ratios des mono-di- et triglycérides étant conservés par rapport au traitement 3, en présence de support (silice) et de terre de diatomées, et
7- Spores traitées avec du Diméthyl sulfoxide (DMSO).

Les concentrations testées sont basées sur le fait d'avoir une valeur iso concernant la teneur en produit, et le cas échéant en terre de diatomées, par rapport au traitement 2.

La composition utilisée comprend :
- 43% d'un mélange de mono, di et triester d'acides gras en C4/C6/C8 comprenant au moins 30% d'acide hexanoïque, et au plus 20% de glycérol,
- 14% de terre de diatomée, et
- 42% de silice
(voir formule 1 exemple 5)

### I.2 - Test de germination des spores

1. Les spores d'EHP utilisées dans cette étude sont purifiées selon le protocole standard BIOTEC (Aldama-Cano et al., 2018).

Des crevettes *Penaeus vannamei* (ayant un poids compris entre 6 et 10 g) fortement infectées par l'EHP sont obtenues à partir de systèmes d'élevages intensifs thaïlandais. Leur niveau d'infection à l'EHP est confirmé par SWP-PCR nichée.

Les crevettes vivantes présentant un niveau d'infection « moyen » à « élevé » sont anesthésiées dans une solution d'eau de mer artificielle glacée (Marinium) avant une dissection minutieuse du tissu hépatopancréatique. Le tissu est homogénéisé avec de l'eau distillée stérile pour obtenir une concentration finale de 10 % (vol/vol) dans un tube Falcon de 50 ml.

Les spores sont séparées de l'homogénat par centrifugation en deux étapes : une centrifugation à basse vitesse à 3500xg pendant 10 min suivie d'une séparation par gradient Percoll (GE Technologies) de densité discontinue à 40 000xg pendant 30 min. Les spores sont collectées et lavées 3 fois avec de l'eau distillée avant stockage à température ambiante (25°C). La concentration des spores est déterminée par comptage cellulaire à l'aide d'un hémocytomètre.

2. Les spores purifiées sont aliquotées et incubées avec les traitements sélectionnés pendant 30 min à température ambiante (27°C) dans des tubes Eppendorf et un volume total de 1ml par réaction.

L'effet des traitements sur la germination et la viabilité des spores est mesuré par les méthodes décrites ci-dessous.

Une solution de 10 µl de Phloxine B à 2 % w/v (CI 45410, Merck-Millipore) dans de l'eau distillée stérilisée est déposée sur une lame de microscope suivi de l'ajout de 2µl de 1,0 × 10⁶ spores purifiées et 10µl de 0,1 % w/v de carboxyméthylcellulose sodique (Sunrose) dans de l'eau distillée stérilisée comme agent stabilisant.

La lame est incubée à température ambiante (25 °C) pendant 10 min avant d'être observée au microscope optique (Leica DM750) sous un objectif 100× avec de l'huile d'immersion (Olympus).

Le taux de germination ou le pourcentage d'extrusion de spores est calculé à partir du nombre de spores extrudées (ou germées) sur les 100 spores observées.

Afin d'évaluer la robustesse des résultats, ce test est réalisé en 3 réplicas et 2 répétitions.

L'analyse statistique est effectuée par un test T avec un intervalle de confiance de 95% pour déterminer s'il existe une différence significative entre les taux de germination.

### II - RESULTATS

Les résultats sont présentés à la **Figure 8****.**

Les contrôles #1 et #7 (respectivement tampon PBS et diluant DMS) sont validés et présentent les germinations de spores les plus élevées (respectivement 82 et 79%). L'absence de traitement ou le solvant n'entrainent donc pas d'inhibition de la germination des spores.

Ceci constitue le niveau basal sans traitement. Naturellement environ 20% des spores ne semblent pas germer.

Les variabilités par traitement sont faibles et indiquent une certaine robustesse des résultats obtenus.

Le meilleur traitement obtenu est celui du traitement 2 correspondant à la composition selon l'invention avec un taux de germination de 20% et donc 80% d'inhibition des spores.

On note en contrôle que la terre de diatomée seule permet une diminution de la germination de 50%, et que le produit de l'invention (acides gras C4/C6/C8 estérifiés dont au moins 30% de C6) permet une diminution de la germination de 64%.

Cela justifie l'intérêt de la combinaison produit et terre de diatomées de la formule.

On note en outre que contrairement à ce qu'enseigne l'art antérieur, un enrichissement en C8 de la composition selon l'invention (traitement 6) n'apporte pas d'amélioration d'efficacité qui au contraire est plus dégradée, avec une diminution de la germination de 62% (contre 80% pour la composition selon l'invention).

En outre, il est à noter que le produit selon l'invention et un produit similaire enrichi en C8 (au détriment du C6) ont des effets similaires sur la germination (traitements 3 et 4).

Aussi ces résultats *in vitro* montrent que la combinaison spécifique de mélange de mono, di et triester d'acides gras comprenant au moins 30% de d'acides gras en C6 en masse par rapport à la masse totale des acides gras, additionné de terre de diatomée, permet une meilleure diminution de la germination par rapport au même produit sans terre de diatomées ou un produit présentant une forte proportion de d'acides gras en C8, et donc exerce un effet antiparasitaire inattendu par rapport à l'art antérieur.

### Exemple 5 - Exemples de compositions selon l'invention

**[Table 2]**

| Formule | 1 | 2 | 3 | 4 | 5 | 6 | 7 | 8 | 9 |
|---|---|---|---|---|---|---|---|---|---|
| Acides gras | 43% | 43% | 43% | 55% | 55% | 55% | 45% | 45% | 45% |
| C4 : | *15,3 %* | *19,8 %* | *22,4 %* | *15,3 %* | *19,8 %* | *22,4 %* | *15,3%* | *19,8%* | *22,4%* |
| C6 : | *64,3 %* | *57,4 %* | *56,1 %* | *64,3 %* | *57,4 %* | *56,1 %* | *64,3%* | *57,4%* | *56,1%* |
| C8 : | *20,4 %* | *22,8 %* | *21,5 %* | *20,4 %* | *22,8 %* | *21,5 %* | *20,4%* | *22,8%* | *21,5%* |
| Support | 14% Silice | 14% Silice | 14% Silice | 30% Silice | 30% Silice | 30% Silice | 30% Sépiolite | 30% Sépiolite | 30% Sépiolite |
| Terre de diatomées | 42% | 42% | 42% | 15% | 15% | 15% | 25% | 25% | 25% |

**[Table 3]**

| Formule | 10 | 11 | 12 | 13 | 14 | 15 | 16 | 17 | 18 |
|---|---|---|---|---|---|---|---|---|---|
| Acides gras | 45% | 45% | 45% | 23% | 23% | 23% | 60% | 60% | 60% |
| C4 : | *15,3 %* | *19,8 %* | *22,4 %* | *15,3%* | *19,8%* | *22,4%* | *15,3%* | *19,8%* | *22,4%* |
| C6 : | *64,3 %* | *57,4 %* | *56,1 %* | *64,3%* | *57,4%* | *56,1%* | *64,3%* | *57,4%* | *56,1%* |
| C8 : | *20,4 %* | *22,8 %* | *21,5 %* | *20,4%* | *22,8%* | *21,5%* | *20,4%* | *22,8%* | *21,5%* |
| Support | 25% Grits de maïs | 25% Grits de maïs | 25% Grits de maïs | 40% Rémoula ge de blé | 40% Rémoula ge de blé | 40% Rémoula ge de blé | 19% Sépioli te | 19% Sépioli te | 19% Sépioli te |
| Terre de diatomées | 30% | 30% | 30% | 37% | 37% | 37% | 18% | 18% | 18% |

## Revendications

1. Composition consistant en :
- de 10 à 50% en masse d'au moins un support, et
- de 35 à 90% en masse d'un produit en association avec une diatomite, ledit produit consistant essentiellement en au moins- un monoglycéride d'un ou plusieurs acide(s) gras, ou
- un diglycéride d'un ou plusieurs acide(s) gras, ou
- un triglycéride d'un ou plusieurs acide(s) gras, ou
- un mélange de ceux-ci,
ledit ou lesdits un ou plusieurs acide(s) gras étant un mélange d'acides gras comportant de 4 à 8 atomes de carbone
les pourcentages étant exprimés en masse par rapport à la masse totale de la composition,
ledit ou lesdits un ou plusieurs acide(s) gras étant composé(s) d'au moins 30% d'acide hexanoïque, ce pourcentage étant exprimé en masse par rapport à la masse totale d'acide gras.

2. Composition selon la revendication 1, ledit ou lesdits un ou plusieurs acide(s) gras étant composé(s) :
- de 5 à 25% d'acide butyrique,
- de 30 à 90% d'acide hexanoïque, et
- de 5 à 45% d'acide octanoïque,
les pourcentages étant exprimés en masse par rapport à la masse totale d'acides gras.

3. Composition selon la revendication 1 ou 2, où ladite diatomite représente de 18 à 50 % en masse par rapport à la masse totale du produit et de ladite diatomite.

4. Composition selon l'une quelconque des revendications 1 à 3, ladite composition étant choisie parmi l'une des dix-huit compositions suivantes :
| composition | **1** | **2** | **3** | **4** | **5** | **6** | **7** | **8** | **9** |
|---|---|---|---|---|---|---|---|---|---|
| Acides gras | 43% | 43% | 43% | 55% | 55% | 55% | 45% | 45% | 45% |
| C4 : | *15,3%* | *19,8%* | *22,4%* | *15,3%* | *19,8%* | *22,4%* | *15,3%* | *19,8%* | *22,4%* |
| C6 : | *64,3%* | *57,4%* | *56,1%* | *64,3%* | *57,4%* | *56,1%* | *64,3%* | *57,4%* | *56,1%* |
| C8 : | *20,4%* | *22,8%* | *21,5%* | *20,4%* | *22,8%* | *21,5%* | *20,4%* | *22,8%* | *21,5%* |
| Support | | | | | | | | | |
|---|---|---|---|---|---|---|---|---|---|
| Silice | 14% | 14% | 14% | 30% | 30% | 30% | - | - | - |
| Sépiolite | - | - | - | - | - | - | 30% | 30% | 30% |
| Grits de maïs | - | - | - | - | - | - | - | - | - |
| Rémoulage de blé | - | - | - | - | - | - | - | - | - |
| Terre de diatomées | 42% | 42% | 42% | 15% | 15% | 15% | 25% | 25% | 25% |
| | | | | | | | | | |
| Composition | **10** | **11** | **12** | **13** | **14** | **15** | **16** | **17** | **18** |
|---|---|---|---|---|---|---|---|---|---|
| Acides gras | 45% | 45% | 45% | 23% | 23% | 23% | 60% | 60% | 60% |
| C4 : | *15,3%* | *19,8%* | *22,4%* | *15,3%* | *19,8%* | *22,4%* | *15,3%* | *19,8%* | *22,4%* |
| C6 : | *64,3%* | *57,4%* | *56,1%* | *64,3%* | *57,4%* | *56,1%* | *64,3%* | *57,4%* | *56,1%* |
| C8 : | *20,4%* | *22,8%* | *21,5%* | *20,4%* | *22,8%* | *21,5%* | *20,4%* | *22,8%* | *21,5%* |
| Support | | | | | | | | | |
| Silice | - | - | - | - | - | - | - | - | - |
| Sépiolite | - | - | - | - | - | - | 19% | 19% | 19% |
| Grits de maïs | 25% | 25% | 25% | - | - | - | - | - | - |
| Rémoulage de blé | - | - | - | 40% | 40% | 40% | - | - | - |
| Terre de diatomées | 30% | 30% | 30% | 37% | 37% | 37% | 18% | 18% | 18% |

5. Composition selon l'une quelconque des revendications 1 à 4, en tant que médicament.

6. Composition selon l'une quelconque des revendications 1 à 4, pour son utilisation pour la prévention et/ou le traitement de pathologies causées par des parasites d'animaux, notamment des animaux d'élevage, notamment des animaux aquatiques d'élevage ou d'oiseaux d'élevage.

7. Procédé de préparation d'une poudre de la composition telle que définie dans l'une quelconque des revendications 1 à 4, ledit procédé comprenant :
le brassage d'un premier, d'un deuxième et d'un troisième ingrédient, pour obtenir une poudre de ladite composition,
1- le premier ingrédient étant un produit liquide consistant essentiellement en au moins
- un monoglycéride d'un ou plusieurs acide(s) gras, ou
- un diglycéride d'un ou plusieurs acide(s) gras, ou
- un triglycéride d'un ou plusieurs acide(s) gras, ou
- un mélange de ceux-ci,
ledit produit représentant de 15 à 70% en masse,
ledit ou lesdits un ou plusieurs acide(s) gras étant composé(s) d'au moins 30% en masse d'acide hexanoïque, ce pourcentage étant exprimé en masse par rapport à la masse totale d'acides gras,
2- le deuxième ingrédient étant une diatomite sous forme de poudre, dans une proportion de 5 à 45% en masse, et
3- le troisième ingrédient étant au moins un support sous forme de poudre dans une proportion de 10 à 50% en masse,
où les pourcentages sont exprimés en masse par rapport à la masse totale des premier, deuxième et troisième ingrédients.

8. Produit alimentaire comprenant de 0,001% à 2%, notamment de 0,002% à 1%, d'une composition telle que définie dans l'une quelconque des revendications 1 à 4, les pourcentages étant exprimés en masse par rapport à la masse totale du produit alimentaire.

## Patentansprüche

1. Zusammensetzung, bestehend aus:
- zu 10 bis 50 Massenprozent mindestens einen Träger, und
- zu 35 bis 90 Massenprozent ein Produkt in Verbindung mit Kieselgur, wobei das Produkt im Wesentlichen aus mindestens einem Monoglycerid einer oder mehrerer Fettsäure(n) besteht oder
- einem Diglycerid einer oder mehrerer Fettsäure(n) oder
- einem Triglycerid einer oder mehrerer Fettsäure(n) oder
- einer Mischung davon,
wobei die eine oder die mehreren Fettsäure(n) eine Mischung von Fettsäuren sind, die von 4 bis 8 Kohlenstoffatome aufweisen
wobei die Prozentsätze in Masse relativ zu der Gesamtmasse der Zusammensetzung ausgedrückt sind,
wobei die eine oder die mehreren Fettsäure(n) zu mindestens 30 % aus Hexansäure bestehen, wobei dieser Prozentsatz in Masse relativ zu der Gesamtmasse der Fettsäure ausgedrückt wird.

2. Zusammensetzung nach Anspruch 1, wobei die eine oder die mehreren Fettsäure(n) zusammengesetzt sind aus:
- 5 bis 25 % Buttersäure,
- 30 bis 90 % Hexansäure, und
- 5 bis 45 % Octansäure,
wobei die Prozentsätze in Masse relativ zu der Gesamtmasse der Fettsäuren ausgedrückt sind.

3. Zusammensetzung nach Anspruch 1 oder 2, wobei der Kieselguranteil 18 bis 50 Massen-% relativ zu der Gesamtmasse des Produkts und des Kieselgurs beträgt.

4. Zusammensetzung nach einem der Ansprüche 1 bis 3, wobei die Zusammensetzung aus einer der folgenden achtzehn Zusammensetzungen ausgewählt ist:
| Zusammensetzung | **1** | **2** | **3** | **4** | **5** | **6** | **7** | **8** | **9** |
|---|---|---|---|---|---|---|---|---|---|
| Fettsäuren | 43 % | 43 % | 43 % | 55 % | 55 % | 55 % | 45 % | 45 % | 45 % |
| C4: | *15,3 %* | *19,8 %* | *22,4 %* | *15,3 %* | *19,8 %* | *22,4 %* | *15,3 %* | *19,8 %* | *22,4 %* |
| C6: | *64,3 %* | *57,4 %* | *56,1 %* | *64,3 %* | *57,4 %* | *56,1 %* | *64,3 %* | *57,4 %* | *56,1 %* |
| C8: | *20,4 %* | *22,8 %* | *21,5 %* | *20,4 %* | *22,8 %* | *21,5 %* | *20,4 %* | *22,8 %* | *21,5 %* |
| Träger | | | | | | | | | |
|---|---|---|---|---|---|---|---|---|---|
| Kieselerde | 14 % | 14 % | 14 % | 30 % | 30 % | 30 % | - | - | - |
| Sepiolith | - | - | - | - | - | - | 30 % | 30 % | 30 % |
| Maisgrieß | - | - | - | - | - | - | - | - | - |
| Weizennachmehl | - | - | - | - | - | - | - | - | - |
| Kieselerde | 42 % | 42 % | 42 % | 15 % | 15 % | 15 % | 25 % | 25 % | 25 % |
| | | | | | | | | | |
| Zusammensetzung | **10** | **11** | **12** | **13** | **14** | **15** | **16** | **17** | **18** |
|---|---|---|---|---|---|---|---|---|---|
| Fettsäuren | 45 % | 45 % | 45 % | 23 % | 23 % | 23 % | 60 % | 60 % | 60 % |
| C4: | *15,3 %* | *19,8 %* | *22,4 %* | *15,3 %* | *19,8 %* | *22,4 %* | *15,3 %* | *19,8 %* | *22,4 %* |
| C6: | *64,3 %* | *57,4 %* | *56,1 %* | *64,3 %* | *57,4 %* | *56,1 %* | *64,3 %* | *57,4 %* | *56,1 %* |
| C8: | *20,4 %* | *22,8 %* | *21,5 %* | *20,4 %* | *22,8 %* | *21,5 %* | *20,4 %* | *22,8 %* | *21,5 %* |
| Träger | | | | | | | | | |
| Kieselerde | - | - | - | - | - | - | - | - | - |
| Sepiolith | - | - | - | - | - | - | 19 % | 19 % | 19 % |
| Maisgrieß | 25 % | 25 % | 25 % | - | - | - | - | - | - |
| Weizennachmehl | - | - | - | 40 % | 40 % | 40 % | - | - | - |
| Kieselerde | 30 % | 30 % | 30 % | 37 % | 37 % | 37 % | 18 % | 18 % | 18 % |

5. Zusammensetzung nach einem der Ansprüche 1 bis 4 als Arzneimittel.

6. Zusammensetzung nach einem der Ansprüche 1 bis 4 zur Verwendung bei der Vorbeugung und/oder der Behandlung von durch Parasiten verursachten Erkrankungen von Tieren, insbesondere Nutztieren, insbesondere Nutzwassertieren oder Nutzvögeln.

7. Verfahren zum Herstellen eines Pulvers der Zusammensetzung nach einem der Ansprüche 1 bis 4, das Verfahren umfassend:
das Mischen eines ersten, eines zweiten und eines dritten Bestandteils zum Erhalten eines Pulver der Zusammensetzung,
1- wobei die erste Zutat ein flüssiges Produkt ist, im Wesentlichen bestehend aus mindestens
- einem Monoglycerid einer oder mehrerer Fettsäure(n) oder
- einem Diglycerid einer oder mehrerer Fettsäure(n) oder
- einem Triglycerid einer oder mehrerer Fettsäure(n) oder
- einer Mischung davon,
wobei das Produkt 15 bis 70 Massen-% ausmacht,
wobei die eine oder die mehreren Fettsäure(n) zu mindestens 30 Massen-% aus Hexansäure besteht/bestehen, wobei dieser Prozentsatz als Massenanteil relativ zu der Gesamtmasse der Fettsäuren ausgedrückt wird,
2- der zweite Bestandteil ein Kieselgur in Pulverform in einem Massenanteil von 5 bis 45 % ist, und
3- der dritte Bestandteil mindestens ein Träger in Pulverform in einem Anteil von 10 bis 50 Massen-% ist,
wobei die Prozentsätze in Masse relativ zu der Gesamtmasse des ersten, des zweiten und des dritten Bestandteils ausgedrückt sind.

8. Nahrungsmittelprodukt, umfassend zu 0,001 % bis 2 %, insbesondere zu 0,002 % bis 1 %, eine Zusammensetzung nach einem der Ansprüche 1 bis 4, wobei die Prozentangaben in Masse bezogen auf die Gesamtmasse des Nahrungsmittelprodukts ausgedrückt sind.

## Claims

1. Composition consisting of:
- from 10 to 50% by weight of at least a support, and
- from 35 to 90% by weight of a product in association with a diatomite, said product consisting essentially of at least
- a monoglyceride of one or more fatty acid(s), or
- a diglyceride of one or more fatty acid(s), or
- a triglyceride of one or more fatty acid(s), or
- a mixture thereof,
Said one or more fatty acid(s) being a mixture of fatty acids comprising from 4 to 8 carbons,
percentages being expressed by weight compared to the total weight of the composition,
said one or more fatty acid(s) comprising at least 30% of hexanoic acid, this percentage being expressed in weight compared to the total weight of fatty acids.

2. Composition according to claim 1, said one or more fatty acid(s) comprising:
- from 5 to 25% of butyric acid,
- from 30 to 90% of hexanoic acid, and
- from 5 to 45% of octanoic acid,
percentages being expressed in weight compared to the total weight of fatty acids.

3. Composition according to claim 1 or 2, wherein said diatomite represents from 18 to 50 % by weight compared to the total weight of the product and said diatomite.

4. Composition according to anyone of claims 1 to 3, said composition being chosen from one of the eighteen following compositions:
| composition | **1** | **2** | **3** | **4** | **5** | **6** | **7** | **8** | **9** |
|---|---|---|---|---|---|---|---|---|---|
| Fatty acid | 43% | 43% | 43% | 55% | 55% | 55% | 45% | 45% | 45% |
| C4 : | *15,3%* | *19,8%* | *22,4%* | *15,3%* | *19,8%* | *22,4%* | *15,3%* | *19,8%* | *22,4%* |
| C6 : | *64,3%* | *57,4%* | *56,1%* | *64,3%* | *57,4%* | *56,1%* | *64,3%* | *57,4%* | *56,1%* |
| C8 : | *20,4%* | *22,8%* | *21,5%* | *20,4%* | *22,8%* | *21,5%* | *20,4%* | *22,8%* | *21,5%* |
| Support | | | | | | | | | |
| Silica | 14% | 14% | 14% | 30% | 30% | 30% | - | - | - |
| Sepiolite | - | - | - | - | - | - | 30% | 30% | 30% |
| Corn grits | - | - | - | - | - | - | - | - | - |
| Wheat middlings | - | - | - | - | - | - | - | - | - |
| Diatomaceous earth | 42% | 42% | 42% | 15% | 15% | 15% | 25% | 25% | 25% |
| | | | | | | | | | |
| Composition | **10** | **11** | **12** | **13** | **14** | **15** | **16** | **17** | **18** |
|---|---|---|---|---|---|---|---|---|---|
| Fatty acid | 45% | 45% | 45% | 23% | 23% | 23% | 60% | 60% | 60% |
| C4 : | *15,3%* | *19,8%* | *22,4%* | *15,3%* | *19,8%* | *22,4%* | *15,3%* | *19,8%* | *22,4%* |
| C6 : | *64,3%* | *57,4%* | *56,1%* | *64,3%* | *57,4%* | *56,1%* | *64,3%* | *57,4%* | *56,1%* |
| C8 : | *20,4%* | *22,8%* | *21,5%* | *20,4%* | *22,8%* | *21,5%* | *20,4%* | *22,8%* | *21,5%* |
| Support | | | | | | | | | |
| Silica | - | - | - | - | - | - | - | - | - |
| Sepiolite | - | - | - | - | - | - | 19% | 19% | 19% |
| Corn grits | 25% | 25% | 25% | - | - | - | - | - | - |
| Wheat middlings | - | - | - | 40% | 40% | 40% | - | - | - |
| Diatomaceous earth | 30% | 30% | 30% | 37% | 37% | 37% | 18% | 18% | 18% |

5. Composition according to anyone of claims 1 to 4, as a drug.

6. Composition according to anyone of claims 1 to 4, for its use for prevention and/or treatment of pathologies caused by animal parasites, in particular farm animals, more particularly aquatic farm animals or farm birds.

7. Process for preparing a powder of the composition as defined in anyone of claims 1 to 4, said process comprising:
mixing a first, second, and third ingredient to obtain a powder of said composition,
1- the first ingredient being a liquid product consisting essentially of at least
- a monoglyceride of one or more fatty acids, or
- a diglyceride of one or more fatty acids, or
- a triglyceride of one or more fatty acids, or
- a mixture thereof,
said product representing 15 to 70% by weight,
said one or more fatty acids being composed of at least 30% by weight of hexanoic acid, this percentage being expressed by weight relative to the total weight of fatty acids,
2- the second ingredient being diatomite in powder form, in a proportion of 5 to 45% by mass, and
3- the third ingredient being at least one carrier in powder form in a proportion of 10 to 50% by mass,
where the percentages are expressed by mass relative to the total mass of the first, second, and third ingredients.

8. Food product comprising from 0.001% to 2%, in particular from 0.002% to 1%, of a composition as defined in anyone of claims 1 to 4, percentages being expressed in weight compared to total weight of the food product.
